(19) Europäisches Patentamt
European Patent Office
Office européen des brevets

(11) EP 0 487 095 B1

(12) # EUROPÄISCHE PATENTSCHRIFT

(45) Veröffentlichungstag und Bekanntmachung des Hinweises auf die Patenterteilung: **28.02.1996 Patentblatt 1996/09**

(51) Int. Cl.$^6$: **C07D 213/55**, C07D 409/12, C07D 401/12, A61K 31/44

(21) Anmeldenummer: **91119889.3**

(22) Anmeldetag: **21.11.1991**

(54) **Neue Pyridylderivate, diese Verbindungen enthaltende Arzneimittel und Verfahren zu ihrer Herstellung**

Pyridyl derivatives, medicines containing these compounds and processes for their preparation

Dérivés de pyridines, médicaments contenant ces composés et procédé pour leur préparation

(84) Benannte Vertragsstaaten:
**AT BE CH DE DK ES FR GB GR IT LI LU NL SE**

(30) Priorität: **22.11.1990 DE 4037112**

(43) Veröffentlichungstag der Anmeldung: **27.05.1992 Patentblatt 1992/22**

(73) Patentinhaber: **Dr. Karl Thomae GmbH
D-88397 Biberach (DE)**

(72) Erfinder:
- **Soyka, Rainer, Dr. Dipl.-Chem.
W-7950 Biberach (DE)**
- **Eisert, Wolfgang, Arzt. Prof. Dr. Dr. Dr.
W-7950 Biberach 1 (DE)**
- **Müller, Thomas, Dr. Arzt und Dipl.-Chem.
W-7950 Biberach 1 (DE)**
- **Weisenberger, Johannes, Dr. Dipl.-Chem.
W-7950 Biberach 1 (DE)**

(56) Entgegenhaltungen:
**EP-A- 0 111 997**
**EP-A- 0 287 270**
**EP-A- 0 397 044**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).



Printed by Rank Xerox (UK) Business Services
2.9.9/3.4

**Beschreibung**

In der EP-A-0,397,044 sowie in der nicht vorveröffentlichten EP-A-0,471,259 werden bereits Arylsulfonamide beschrieben, welche antithrombotische Eigenschaften aufweisen.

Es wurde nun gefunden, daß die neuen Pyridylderivate der allgemeinen Formel

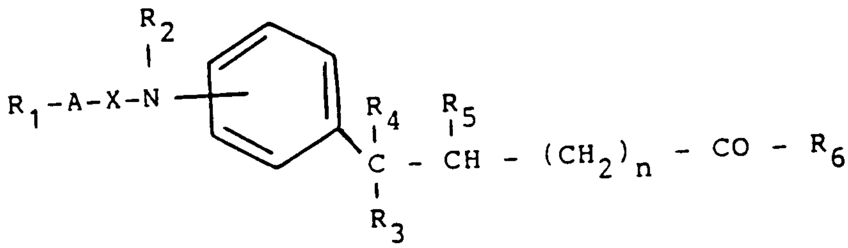

,(I)

welche keine Sulfonylgruppe enthalten, deren Enantiomere, deren cis- und trans-Isomere, sofern $R_4$ und $R_5$ zusammen eine Kohlenstoff-Kohlenstoff-Bindung darstellen, sowie deren Additionssalze, insbesondere für die pharmazeutische Anwendung deren physiologisch verträgliche Additionssalze mit anorganischen oder organischen Basen, falls $R_6$ eine Hydroxygruppe darstellt, wertvolle pharmakologische Eigenschaften aufweisen, insbesondere antithrombotische Wirkungen. Außerdem stellen die neuen Verbindungen gleichzeitig Thromboxanantagonisten (TRA) und Thromboxansynthesehemmer (TSH) dar und inhibieren somit auch die durch Thromboxan vermittelten Wirkungen. Ferner weisen diese auch eine Wirkung auf die $PGE_2$-Produktion in der Lunge und auf die $PGD_2$-, $PGE_2$- und $PGF_{2\alpha}$-Produktion in Humanthrombozyten auf.

Gegenstand der vorliegenden Erfindung sind somit die neuen Verbindungen der obigen Formel I, deren Additionssalze mit anorganischen oder organischen Basen, insbesondere für die pharmazeutische Verwendung deren physiologisch verträgliche Additionssalze, diese Verbindungen enthaltende Arzneimittel und Verfahren zu ihrer Herstellung.

In der obigen allgemeinen Formel bedeutet

n die Zahl 2, 3 oder 4,

X eine Carbonyl- oder Thiocarbonylgruppe,

$R_1$ eine gegebenenfalls durch eine Phenylgruppe substituierte Alkylgruppe mit 1 bis 4 Kohlenstoffatomen, eine Cycloalkylgruppe mit 4 bis 7 Kohlenstoffatomen, eine Naphthyl-, Biphenylyl-, Diphenylmethyl-, Indolyl-, Thienyl-, Chlorthienyl- oder Bromthienylgruppe, eine Phenylgruppe oder auch, wenn A keine Bindung darstellt, eine Benzoylgruppe, in denen der Phenylkern jeweils durch Fluor-, Chlor- oder Bromatome, Alkoxy- oder Alkylgruppen mit 1 bis 4 Kohlenstoffatomen mono-, di- oder trisubstituiert sein kann, wobei die Substituenten gleich oder verschieden sein können und einer der Substituenten auch eine Trifluormethyl-, Carboxyl-, Amino- oder Nitrogruppe darstellen kann,

$R_2$ ein Wasserstoffatom oder eine Alkylgruppe mit 1 bis 4 Kohlenstoffatomen,

$R_3$ eine Pyridylgruppe,

$R_4$ und $R_5$ jeweils ein Wasserstoffatom oder zusammen eine weitere Kohlenstoff-Kohlenstoff-Bindung,

$R_6$ eine Hydroxy- oder Alkoxygruppe mit 1 bis 3 Kohlenstoffatomen und

A eine Bindung, eine Cycloalkylen- oder Cycloalkylidengruppe mit jeweils 3 oder 4 Kohlenstoffatomen, in denen eine Methylengruppe durch eine Dichlormethylengruppe ersetzt sein kann, eine geradkettige gegebenenfalls ein- oder mehrfach ungesättigte Alkylengruppe oder eine Oxyalkylengruppe mit 2 oder 3 Kohlenstoffatomen, eine $-R_7CR_8-$, $-O-R_7CR_8-$, $-NR_9-$ oder $-XNR_9-$Gruppe, in denen

X wie vorstehend erwähnt definiert ist,

$R_7$ ein Wasserstoffatom, eine Hydroxy-, Phenyl- oder Alkylgruppe mit 1 bis 3 Kohlenstoffatomen,

$R_8$ ein Wasserstoffatom oder eine Alkylgruppe mit 1 bis 3 Kohlenstoffatomen und

$R_9$ ein Wasserstoffatom, eine Alkylgruppe mit 1 bis 3 Kohlenstoffatomen oder eine Phenylgruppe darstellen.

Für die bei der Definition der Reste eingangs erwähnten Bedeutungen kommt beispielsweise für

$R_1$ die der Methyl-, Ethyl-, n-Propyl-, Isopropyl-, n-Butyl-, tert.-Butyl-, Benzyl-, 2-Phenylethyl-, 3-Phenylpropyl-, Cyclobutyl-, Cyclopentyl-, Cyclohexyl-, Phenyl-, Benzoyl-, Benzolsulfonyl-, Fluorphenyl-, Fluorbenzoyl-, Fluorbenzolsulfonyl-, Chlorphenyl-, Chlorbenzoyl-, Bromphenyl-, Brombenzoyl-, Methylphenyl-, Methylbenzoyl-, Ethylphenyl-, Ethylbenzoyl-, n-Propylphenyl-, n-Propylbenzoyl-, Isopropylphenyl-, Isopropylbenzoyl-, n-Butylphenyl-, n-Butylbenzoyl-, Isobutylphenyl-, Isobutylbenzoyl-, tert.Butylphenyl-, tert.Butylbenzoyl-, Trifluormethylphenyl-, Trifluormethylbenzoyl-, Nitrophenyl-, Nitrobenzoyl-, Aminophenyl-, Aminobenzoyl-, Carboxyphenyl-, Carboxybenzoyl-, Methoxyphenyl-, Methoxybenzoyl-, Ethoxy- phenyl-, Ethoxybenzoyl-, Isopropoxyphenyl-, Isopropoxybenzoyl-, Difluorphenyl-, Difluorbenzoyl-, Dichlorphenyl-, Dichlorbenzoyl-, Dimethylphenyl-, Dimethylbenzoyl-, Dimethoxyphenyl-, Dimethoxybenzoyl-, Trimethylphenyl-, Trimethylbenzoyl-, Trimethoxyphenyl-, Trimethoxybenzoyl-, Chlor-methylphenyl-, Chlor-methylbenzoyl-, Chlor-methoxyphenyl-, Chlor-methoxybenzoyl-, Methoxy-methylphenyl-, Methoxy-methylbenzoyl-, Amino-dichlorphenyl-, Amino-dichlorbenzoyl-, Amino-dibromphenyl-, Amino-dibrombenzoyl-, Naphthyl-, Biphenyl-, Biphenylmethyl-, Indolyl-, Thienyl-, Chlorthienyl- oder Bromthienylgruppe,
für $R_2$ die des Wasserstoffatoms, der Methyl-, Ethyl-, n-Propyl-, Isopropyl-, n-Butyl-, Isobutyl- oder tert.Butylgruppe,
für $R_3$ die der Pyridyl-(2)-, Pyridyl-(3)- oder Pyridyl-(4)-gruppe,
$R_6$ die der Hydroxy-, Methoxy-, Ethoxy-, n-Propoxy- oder Isopropoxygruppe und
für die -A-X-Gruppe die der Carbonyl-, Methylencarbonyl-, Ethylencarbonyl-, n-Propylencarbonyl-, Ethenylencarbonyl-, Ethinylencarbonyl-, 1,1-Cyclopropylencarbonyl-, 1,2-Cyclopropylencarbonyl-, 3,3-Dichlor-1,1-cyclopropylen- carbonyl-, 3,3-Dichlor-1,2-cyclopropylencarbonyl-, 1,1-Cyclobutylencarbonyl-, 1,2-Cyclobutylencarbonyl-, Carbonyl-1,2-cyclopropylen-carbonyl-, Carbonyl-1,2-cyclobutylen-carbonyl-, Oxymethylencarbonyl-, Oxy-methylmethylencarbonyl-, Oxy-dimethylmethylencarbonyl-, Oxy-n-ethylencarbonyl-, Oxy-n-propylencarbonyl-, Hydroxymethylencarbonyl-, Aminocarbonyl-, Methylaminocarbonyl-, Ethylaminocarbonyl-, n-Propylaminocarbonyl-, Isopropylaminocarbonyl-, n-Butylaminocarbonyl-, Isobutylaminocarbonyl-, Phenylaminocarbonyl-, Aminothiocarbonyl- oder Carbonylaminocarbonylgruppe, wobei die genannte Carbonylgruppe mit der $-NR_2$-Gruppe verknüpft ist, in Betracht.

Bevorzugte Verbindungen der obigen Formel I sind jedoch diejenigen, in der
n die Zahl 2, 3 oder 4,
X eine Carbonyl- oder Thiocarbonylgruppe,
$R_1$ eine Phenylgruppe oder auch, wenn A keine Bindung darstellt, eine Benzoylgruppe, in denen der Phenylkern jeweils durch ein Fluor-, Chlor- oder Bromatom, durch eine Trifluormethyl-, Phenyl-, Methoxy-, Carboxy- oder Nitrogruppe oder durch eine Alkylgruppe mit 1 bis 4 Kohlenstoffatomen monosubstituiert sein kann, eine Phenylgruppe oder auch, wenn A keine Bindung darstellt, eine Benzoylgruppe, in denen der Phenylkern jeweils durch Chlor- oder Bromatome oder durch Methylgruppen disubstituiert ist, wobei die Substituenten gleich oder verschieden sein können, eine Cyclohexyl-, Benzyl-, 4-Amino-3,5-dichlorphenyl-, 4-Amino-3,5-dibromphenyl-, Naphthyl-, Diphenylmethyl-, Indolyl-, Thienyl-, Chlorthienyl- oder Bromthienylgruppe,
$R_2$ ein Wasserstoffatom oder eine Alkylgruppe mit 1 bis 3 Kohlenstoffatomen,
$R_3$ eine Pyridylgruppe,
$R_4$ und $R_5$ jeweils ein Wasserstoffatom oder zusammen eine weitere Kohlenstoff-Kohlenstoff-Bindung,
$R_6$ eine Hydroxy- oder Alkoxygruppe mit 1 bis 3 Kohlenstoffatomen und
A eine Bindung, eine Cyclopropylen- oder Cyclopropylidengruppe, in denen eine Methylengruppe durch eine Dichlormethylengruppe ersetzt sein kann, eine geradkettige gegebenenfalls ein- oder mehrfach ungesättigte Alkylengruppe mit 2 Kohlenstoffatomen, eine $-R_7CR_8-$, $-O-R_7CR_8-$ oder $-NR_9$-Gruppe, in denen

$R_7$ ein Wasserstoffatom, eine Hydroxy- oder Alkylgruppe mit 1 oder 2 Kohlenstoffatomen,
$R_8$ ein Wasserstoffatom oder eine Alkylgruppe mit 1 oder 2 Kohlenstoffatomen und
$R_9$ ein Wasserstoffatom, eine Alkylgruppe mit 1 oder 2 bis 3 Kohlenstoffatomen oder eine Phenylgruppe darstellen,

bedeuten, deren Enantiomere, deren cis- und trans-Isomeren, sofern $R_4$ und $R_5$ zusammen eine Kohlenstoff-Kohlenstoff-Bindung bilden, sowie deren Additionssalze mit anorganischen oder organischen Basen.

Besonders bevorzugt sind jedoch diejenigen Verbindungen der Formel I, in der
n die Zahl 2, 3 oder 4,
X eine Carbonyl- oder Thiocarbonylgruppe,
$R_1$ eine gegebenenfalls durch ein Fluor-, Chlor- oder Bromatom, durch eine Trifluormethyl-, Phenyl-, Methoxy-, Carboxy- oder Nitrogruppe oder durch eine Alkylgruppe mit 1 bis 4 Kohlenstoffatomen monosubstituierte Phenylgruppe, eine durch Chlor- oder Bromatome oder durch Methylgruppen disubstituierte Phenylgruppe, wobei die Substituenten gleich oder verschieden sein können, eine Benzyl-, 4-Amino-3,5-dichlorphenyl-, Naphthyl- oder Chlorthienylgruppe,
$R_2$ ein Wasserstoffatom oder eine Methylgruppe,
$R_3$ eine 3-Pyridylgruppe,
$R_4$ und $R_5$ jeweils ein Wasserstoffatom oder zusammen eine weitere Kohlenstoff-Kohlenstoff-Bindung,
$R_6$ eine Hydroxygruppe und
A eine Bindung, eine Cyclopropylen- oder Cyclopropylidengruppe, eine geradkettige gegebenenfalls ein- oder mehrfach ungesättigte Alkylengruppe mit 2 Kohlenstoffatomen, eine $-R_7CR_8-$, $-O-R_7CR_8-$ oder $-NR_9$-Gruppe, in denen

$R_7$ ein Wasserstoffatom, eine Hydroxy- oder Methylgruppe,
$R_8$ ein Wasserstoffatom oder eine Methylgruppe und
$R_9$ ein Wasserstoffatom, eine Methyl- oder Phenylgruppe darstellen, bedeuten,

deren Enantiomere, deren cis- und trans-Isomeren, sofern $R_4$ und $R_5$ zusammen eine Kohlenstoff-Kohlenstoff-Bindung bilden, sowie deren physiologisch verträgliche Additionssalze mit anorganischen oder organischen Basen.

Erfindungsgemäß erhält man die neuen Verbindungen nach folgenden Verfahren:

a) Acylierung einer Verbindung der allgemeinen Formel

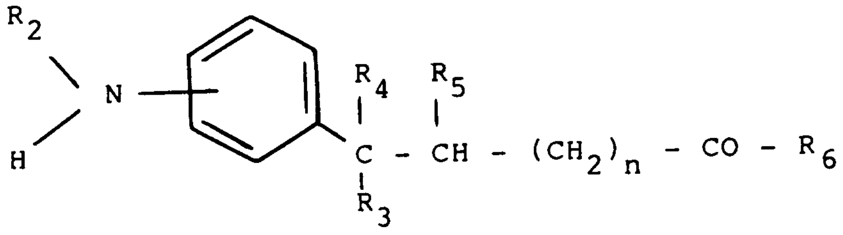

,(II)

in der
$R_2$ bis $R_6$ und n wie eingangs definiert sind, mit einer Verbindung der allgemeinen Formel

$$R_1 - A - X - Z_1 \qquad ,(III)$$

in der
$R_1$ wie eingangs definiert ist und
$Z_1$ eine nucleophile Austrittsgruppe wie ein Halogenatom oder eine Alkoxygruppe, z.B. ein Chlor- oder Bromatom, eine Methoxy- oder Äthoxygruppe, oder auch, wenn A eine -$NR_9$-Gruppe und X eine Carbonyl- oder Thiocarbonylgruppe darstellen, $Z_1$ zusammen mit $R_9$ eine weitere Kohlenstoff-Stickstoff-Bindung darstellt.

Die Umsetzung wird vorzugsweise in einem Lösungsmittel wie Methanol, Äthanol, Wasser/Methanol, Dioxan, Tetrahydrofuran oder Chloroform gegebenenfalls in Gegenwart eines säurebindenden Mittels wie Kaliumcarbonat, Triäthylamin oder Pyridin, wobei die beiden letzteren auch als Lösungsmittel verwendet werden können, zweckmäßigerweise bei Temperaturen zwischen 0 und 50°C, vorzugsweise jedoch bei Raumtemperatur, durchgeführt.

b) Zur Herstellung von Verbindungen der allgemeinen Formel I, in der $R_6$ eine Hydroxygruppe darstellt:
Abspaltung eines Schutzrestes von einer Verbindung der allgemeinen Formel

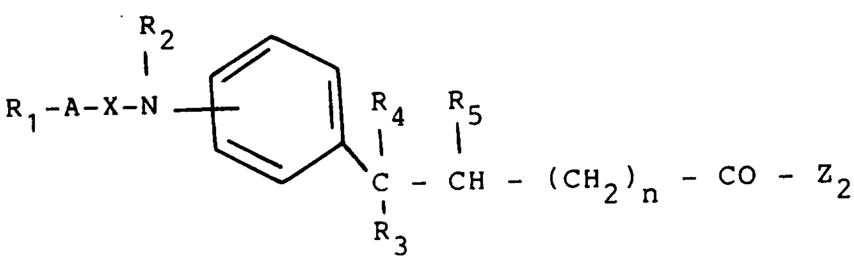

,(IV)

in der
$R_1$ bis $R_5$, A, X und n wie eingangs definiert sind und
$Z_2$ eine hydrolytisch, thermolytisch oder hydrogenolytisch abspaltbare Schutzgruppe für eine Carboxygruppe oder ein funktionelles Derivat der Carboxygruppe darstellt.

Als hydrolysierbare Gruppen kommen beispielsweise funktionelle Derivate der Carboxygruppe wie deren unsubstituierte oder substituierte Amide, Ester, Thioester, Orthoester, Iminoäther, Amidine oder Anhydride, die Nitrilgruppe, Äthergruppen wie die Methoxy-, Ethoxy-, tert.Butoxy- oder Benzyloxygruppe oder Lactone und

als thermolytisch abspaltbare Gruppen beispielsweise Ester mit tertiären Alkoholen, z.B. der tert.Butylester, und als hydrogenolytisch abspaltbare Gruppen beispielsweise Aralkylgruppen, z.B. die Benzylgruppe, in Betracht.

Die Hydrolyse wird zweckmäßigerweise entweder in Gegenwart einer Säure wie Salzsäure, Schwefelsäure, Phosphorsäure oder Trichloressigsäure oder in Gegenwart einer Base wie Natriumhydroxid oder Kaliumhydroxid in einem geeigneten Lösungsmittel wie Wasser, Wasser/Methanol, Äthanol, Wasser/ Äthanol, Wasser/Isopropanol oder Wasser/Dioxan bei Temperaturen zwischen -10 und 120°C, z.B. bei Temperaturen zwischen Raumtemperatur und der Siedetemperatur des Reaktionsgemisches, durchgeführt.

Enthält beispielsweise eine Verbindung der Formel IV eine Nitril- oder Aminocarbonylgruppe, so können diese Gruppen vorzugsweise mittels 100%iger Phosphorsäure bei Temperaturen zwischen 100 und 180°C, vorzugsweise bei Temperaturen zwischen 120 und 160°C, oder auch mit einem Nitrit, z.B. Natriumnitrit, in Gegenwart einer Säure

wie Schwefelsäure, wobei diese zweckmäßigerweise gleichzeitig als Lösungsmittel verwendet wird, bei Temperaturen zwischen 0 und 50°C in die Carboxygruppe übergeführt werden.

Enthält beispielsweise eine Verbindung der Formel IV eine Säureamidgruppe wie die Diethylaminocarbonyl- oder Piperidinocarbonylgruppe, so kann diese Gruppe vorzugsweise hydrolytisch in Gegenwart einer Säure wie Salzsäure, Schwefelsäure, Phosphorsäure oder Trichloressigsäure oder in Gegenwart einer Base wie Natriumhydroxid oder Kaliumhydroxid in einem geeigneten Lösungsmittel wie Wasser, Wasser/Methanol, Äthanol, Wasser/Äthanol, Wasser/Isopropanol oder Wasser/Dioxan bei Temperaturen zwischen -10 und 120°C, z.B. bei Temperaturen zwischen Raumtemperatur und der Siedetemperatur des Reaktionsgemisches, in die Carboxygruppe übergeführt werden.

Enthält beispielsweise eine Verbindung der Formel IV die tert.Butyloxycarbonylgruppe, so kann die tert.Butylgruppe auch thermisch gegebenenfalls in einem inerten Lösungsmittel wie Methlenchlorid, Chloroform, Benzol, Toluol, Tetrahydrofuran oder Dioxan und vorzugsweise in Gegenwart einer katalytischen Menge einer Säure wie p-Toluolsulfonsäure, Schwefelsäure, Phosphorsäure oder Polyphosphorsäure vorzugsweise bei der Siedetemperatur des verwendeten Lösungsmittels, z.B. bei Temperaturen zwischen 40 und 100°C, abgespalten werden.

Enthält beispielsweise eine Verbindung der Formel IV die Benzyloxy- oder Benzyloxycarbonylgruppe, so kann die Benzylgruppe auch hydrogenolytisch in Gegenwart eines Hydrierungskatalysators wie Palladium/Kohle in einem geeigneten Lösungsmittel wie Methanol, Äthanol, Methanol/Wasser, Äthanol/Wasser, Eisessig, Essigsäureäthylester, Dioxan oder Dimethylformamid vorzugsweise bei Temperaturen zwischen 0 und 50°C, z.B. bei Raumtemperatur und einem Wasserstoffdruck von 1 bis 5 bar abgespalten werden. Bei der Hydrogenolyse kann gleichzeitig eine halogenhaltige Verbindung enthalogeniert und eine vorhandende Doppelbindung aufhydriert werden.

c) Zur Herstellung von Verbindungen der allgemeinen Formel I, in der $R_4$ und $R_5$ jeweils ein Wasserstoffatom darstellen:
Hydrierung einer Verbindung der allgemeinen Formel

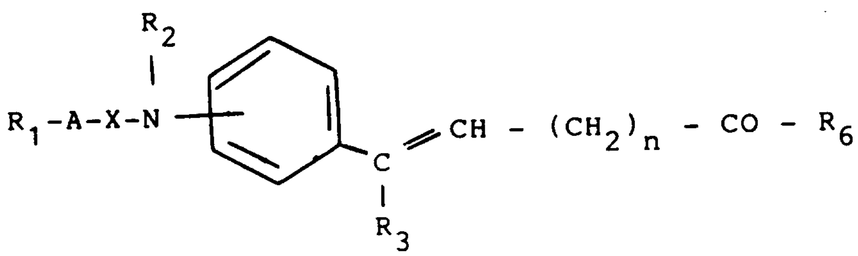

, (V)

in der
$R_1$ bis $R_3$, $R_6$, A, X und n wie eingangs definiert sind.

Die Hydrierung wird in einem geeigneten Lösungsmittel wie Methanol, Äthanol, Dioxan, Essigester oder Eisessig mit katalytisch angeregtem Wasserstoff, z.B. mit Wasserstoff in Gegenwart eines Hydrierungskatalysators wie Raney-Nickel, Palladium, Palladium/Kohle, Platin oder Platin/Kohle und einem Wasserstoffdruck von 1 bis 5 bar, oder mit nascierendem Wasserstoff, z.B. in Gegenwart von Eisen/Salzsäure, Zink/Eisessig, Zinn(II)chlorid/Salzsäure oder Eisen(II)sulfat/Schwefelsäure, bei Temperaturen zwischen 0 und 50°C, vorzugsweise bei Raumtemperatur, durchgeführt. Die katalytische Hydrierung kann jedoch auch stereoselektiv in Gegenwart eines geeigneten Katalysators erfolgen.

Hierbei kann eine gegebenenfalls im Rest $R_1$ vorhandene Nitrogruppe gleichzeitig mitreduziert oder ein gegebenenfalls im Rest $R_1$ vorhandenes Chlor- oder Bromatom durch ein Wasserstoffatom ersetzt werden.

d) Zur Herstellung von Verbindungen der allgemeinen Formel I, in der $R_4$ und $R_5$ zusammen eine Kohlenstoff-Kohlenstoff-Bindung darstellen:

Umsetzung einer Verbindung der allgemeinen Formel

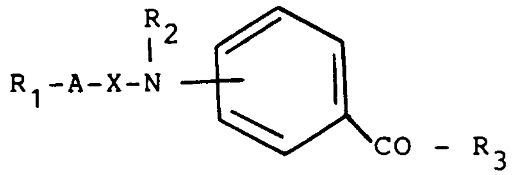

,(VI)

in der
$R_1$ bis $R_3$, A und X wie eingangs definiert sind, mit einer Verbindung der allgemeinen Formel

$$W - CH_2 - (CH_2)_n - CO - R_6 \qquad ,(VII)$$

in der
$R_6$ und n wie eingangs definiert sind und
W einen Triphenylphosphoniumhalogenid-, Dialkylphosphonsäure- oder ein Magnesiumhalogenidrest bedeuten, und gegebenenfalls anschließende Dehydratisierung.

Die Umsetzung wird vorzugsweise in einem geeigneten Lösungsmittel wie Diethylether, Tetrahydrofuran, Dioxan oder Dimethylformamid bei Temperaturen zwischen -30 und 100°C, vorzugsweise bei Temperaturen zwischen -20 und 25°C, durchgeführt.

Die Umsetzung mit einem Triphenylphosphoniumhalogenid der Formel VII wird besonders vorteilhaft jedoch in Gegenwart einer Base wie Kalium-tert.butylat oder Natriumhydrid durchgeführt.

Sollte bei der Umsetzung mit einem Magnesiumhalogenid der Formel VII bei dem im Reaktionsgemisch primär entstandenen Carbinol die Hydroxygruppe nicht während der Umsetzung abgespalten werden, so wird diese in Gegenwart einer Säure wie Salzsäure, Schwefelsäure, Phosphorsäure oder Trichloressigsäure oder in Gegenwart einer Base wie Natriumhydroxid oder Kaliumhydroxid in einem geeigneten Lösungsmittel wie Ethanol, Isopropanol oder Dioxan bei Temperaturen zwischen 0 und 120°C, z.B. bei Temperaturen zwischen Raumtemperatur und der Siedetemperatur des Reaktionsgemisches, abgespalten.

Erhält man erfindungsgemäß eine Verbindung der Formel I, in der $R_2$ ein Wasserstoffatom darstellt, so kann diese mittels Alkylierung in eine entsprechende Verbindung der Formel I, in der $R_2$ eine Alkylgruppe darstellt, übergeführt werden oder
eine Verbindung der Formel I, in der $R_6$ eine Hydroxygruppe darstellt, so kann diese mittels Veresterung in eine entsprechende Verbindung der Formel I, in der $R_6$ eine Alkoxygruppe darstellt, übergeführt werden.

Die nachträgliche Alkylierung wird vorzugsweise in einem Lösungsmittel wie Methylenchlorid, Tetrahydrofuran, Dimethylformamid oder Dimethylsulfoxid in Gegenwart eines Alkylierungsmittels wie Methyljodid, Dimethylsulfat, Ethylbromid, n-Propylbromid oder Isopropylbromid gegebenenfalls in Gegenwart eines säurebindenden Mittels wie Kaliumcarbonat bei Temperaturen zwischen 0 und 70°C, vorzugsweise bei Temperaturen zwischen 20 und 50°C, durchgeführt.

Die nachträgliche Veresterung oder Amidierung wird zweckmäßigerweise in einem Lösungsmittel, z.B. in einem Überschuß des eingesetzten Alkohols wie Methanol, Äthanol oder Isopropanol oder des eingesetzten Amins wie Ammoniak, Methylamin, n-Propylamin oder Dimethylamin, in Gegenwart eines die Säure aktivierenden Mittels wie Thionylchlorid oder Chlorwasserstoffgas bei Temperaturen zwischen 0 und 180°C, vorzugsweise jedoch bei der Siedetemperatur des Reaktionsgemisches, durchgeführt.

Die erhaltenen Verbindungen der Formel I können ferner in ihre Enantiomeren aufgetrennt werden. So lassen sich die erhaltenen Verbindungen der Formel I, welche nur ein optisch aktives Zentrum enthalten, nach an sich bekannten Methoden (siehe Allinger N. L. und Eliel W. L. in "Topics in Stereo-chemistry", Vol. 6, Wiley Interscience, 1971) in ihre optischen Antipoden auftrennen, z.B. durch Umkristallisieren aus einem optisch aktiven Lösungsmittel oder durch Umsetzen mit einer, mit der racemischen Verbindung Salze bildenden optisch aktiven Substanz, insbesondere Basen, und Trennen des auf diese Weise erhaltenen Salzgemisches, z.B. auf Grund von verschiedenen Löslichkeiten, in die diastereomeren Salze, aus denen die freien Antipoden durch Einwirkung geeigneter Mittel freigesetzt werden können. Besonders gebräuchliche, optisch aktive Basen sind z.B. die D- und L-Formen von $\alpha$-Phenyl-äthylamin oder Cinchonidin.

Desweiteren lassen sich die erhaltenen Verbindungen der Formel I mit mindestens 2 asymmetrischen Kohlenstoffatomen auf Grund ihrer physikalischen-chemischen Unterschiede nach an sich bekannten Methoden, z.B. durch Chromatographie und/oder fraktionierte Kristallisation, in ihre Diastereomeren auftrennen. Ein so erhaltenes Enantiomerenpaar läßt sich anschließend in seine optischen Antipoden, wie oben beschrieben, auftrennen. Enthält

beispielsweise eine Verbindung der Formel I zwei optisch aktive Kohlenstoffatome, so erhält man die entsprechenden (R R', S S')- und (R S', S R')-Formen.

Außerdem lassen sich die so erhaltenen Verbindungen der Formel I, in denen $R_4$ und $R_5$ zusammen eine Kohlenstoff-Kohlenstoff-Bindung darstellen, mittels üblichen Methoden beispielsweise durch Chromatographie an einem Träger wie Kieselgel oder durch Kristallisation in ihre cis- und trans-Isomere überführen.

Desweiteren lassen sich die so erhaltenen neuen Verbindungen der Formel I, falls diese eine Carboxygruppe enthalten, gewünschtenfalls anschließend in ihre Additionssalze mit anorganischen oder organischen Basen, insbesondere für die pharmazeutische Anwendung in ihre physiologisch verträglichen Additionssalze, überführen. Als Basen kommen hierbei beispielsweise Natriumhydroxid, Kaliumhydroxid, Cyclohexylamin, Äthanolamin, Diäthanolamin und Triäthanolamin in Betracht.

Die als Ausgangsstoffe verwendeten Verbindungen der Formeln II bis VII erhält man nach literaturbekannten Verfahren bzw. sind literaturbekannt.

Eine als Ausgangsstoff verwendete Verbindung der Formel II erhält man aus einer entsprechenden N-Acylaminoverbindung durch Acylierung nach Friedel-Craft, anschließende Entacylierung und gegebenenfalls anschließende Reduktion, Hydrolyse und/oder Veresterung oder
durch Umsetzung einer entsprechenden Magnesium- oder Lithiumverbindung mit einer entsprechend substituierten Pyridinverbindung wie 3-Cyano-pyridin, Pyridin-3-aldehyd oder einem Pyridin-3-carbonsäurederivat und gegebenenfalls anschließender Oxidation.

Die als Ausgangsstoffe verwendeten Verbindungen der Formeln IV, V und VI erhält man durch Umsetzung einer entsprechenden Aminoverbindung mit einem entsprechenden Halogenid.

Die als Ausgangsstoffe verwendeten Verbindungen der Formel VII erhält man durch Umsetzung einer entsprechenden Halogencarbonsäure mit Triphenylphosphin oder mit einem Trialkylphosphonester.

Wie bereits eingangs erwähnt, weisen die neuen Verbindungen und deren physiologisch verträgliche Additionssalze mit anorganischen oder organischen Basen wertvolle pharmakologische Eigenschaften auf, insbesondere antithrombotische Wirkungen und eine Hemmwirkung auf die Plättchenaggregation. Außerdem stellen sie auch Thromboxanantagonisten und Thromboxansynthesehemmer dar, wobei besonders bemerkenswert ist, daß die Verbindungen der Formel I diese Wirkung gleichzeitig aufweisen. Ferner weisen diese auch eine Wirkung auf die $PGE_2$-Produktion in der Lunge und auf die $PGD_2$-, $PGE_2$- und $PGF_{2\alpha}$-Produktion in Humanthrombozyten auf.

Beispielsweise werden die neuen Verbindungen

A = (-)-5E-6-[4-(Z-2-(4-Chlorphenyl)-cyclopropyl-1-carboxamido)-phenyl]-6-(3-pyridyl)-hex-5-ensäure,

B = 5E-6-[3-(3-(4-Chlorphenyl)-propionylamino)-phenyl]-6-(3-pyridyl)-hex-5-ensäure,

C = 5E-6-[3-(3-(4-Chlorphenyl)-thioureido)-phenyl]-6-(3-pyridyl)-hex-5-ensäure und

D = 5E-6-[3-(3-(2,4-Dichlorphenyl)-1-methylthioureido)-phenyl]-6-(3-pyridyl)-hex-5-ensäure

auf ihre biologischen Eigenschaften wie folgt geprüft:

1. <u>Antithrombotische Wirkung</u>

<u>Methodik</u>

Die Thrombozytenaggregation wird nach der Methode von Born und Cross (J. Physiol. <u>170</u>, 397 (1964)) in plättchenreichem Plasma gesunder Versuchspersonen gemessen. Zur Gerinnungshemmung wird das Blut mit Natriumcitrat 3,14 % im Volumenverhältnis 1:10 versetzt.

<u>Collagen-induzierte Aggregation</u>

Der Verlauf der Abnahme der optischen Dichte der Plättchensuspension wird nach Zugabe der aggregationsauslösenden Substanz photometrisch gemessen und registriert. Aus dem Neigungswinkel der Dichtekurve wird auf die Aggregationsgeschwindigkeit geschlossen. Der Punkt der Kurve, bei dem die größte Lichtdurchlässigkeit vorliegt, dient zur Berechnung der "optical density".

Die Collagen-Menge wird möglichst gering gewählt, aber doch so, daß sich eine irreversibel verlaufende Reaktionskurve ergibt. Verwendet wird das handelsübliche Collagen der Firma Hormonchemie, München.
Vor der Collagen-Zugabe wird das Plasma jeweils 10 Minuten mit der Substanz bei 37°C inkubiert.

Aus den erhaltenen Meßzahlen wird graphisch eine $EC_{50}$ bestimmt, die sich auf eine 50%ige Änderung der "optical density" im Sinne einer Aggregationshemmung bezieht.

2. Thromboxanantagonistische Wirkung

Venöses Humanblut wird mit 13 mM Na3-Citrat antikoaguliert und 10 Minuten bei 170 x g zentrifugiert. Das überstehende plättchenreiche Plasma wird zur Entfernung der Plasmaproteine über eine Sepharose-2B-Säule gegeben. Aliquote der erhaltenen Plättchensuspension werden mit der Testsubstanz, dem Liganden (3H-markiert) und einem Marker (14C-markiert) 60 Minuten bei Raumtemperatur inkubiert und danach 20 Sekunden bei 10 000 x g abzentrifugiert. Der Überstand wird abgezogen und das Pellet in NaOH gelöst. Die 3H-Aktivität im Überstand entspricht dem freien Liganden, 14C ergibt die Konzentration des Markers. 3H im Pellet entspricht dem gebundenen Liganden, 14C wird zur Korrektur für Ligand im Extrazellulärraum verwendet. Aus den Bindungswerten für verschiedene Konzentrationen der Testsubstanz wird nach Iteration die Verdrängungskurve ermittelt und die $IC_{50}$ bestimmt.

3. Bestimmung der Hemmwirkung auf die Thromboxansynthetase

Venöses Humanblut wird mit 13 mM Na3-Citrat antikoaguliert und 10 Minuten bei 170 x g zentrifugiert. Das überstehende plättchenreiche Plasma wird zur Entfernung der Plasmaproteine über eine Sepharose-2B-Säule gegeben. Aliquots der erhaltenen Plättchensuspension werden mit der Testsubstanz bzw. Lösungsmittel als Kontrolle 10 Minuten bei Raumtemperatur inkubiert und nach Zugabe 14C-markierter Arachidonsäure die Inkubation weitere 10 Minuten fortgesetzt. Nach Abstoppen mit 50 µl Zitronensäure wird 3 x mit 500 µl Essigester extrahiert und die vereinigten Extrakte mit Stickstoff abgeblasen. Der Rückstand wird in Essigester aufgenommen, auf DC-Folie aufgetragen und mit Chloroform:Methanol:Eisessig:Wasser (90:8:1:0,8, v/v/v/v) getrennt. Die getrockneten DC-Folien werden 3 Tage auf Röntgenfilm gelegt, die Autoradiogramme entwickelt und mit ihrer Hilfe die aktiven Zonen auf der Folie markiert. Nach Ausschneiden wird die Aktivität im Szintillationszähler bestimmt und die Hemmung der TXB2-Bildung berechnet. Die $IC_{50}$ wird durch lineare Interpolation bestimmt.

Die nachfolgende Tabelle enthält die gefundenen Werte:

| Beispiel | Hemmung der Thromboxansynthetase $IC_{50}$ | Thromboxanantagonistische Wirkung $IC_{50}$ | Hemmung der collagen-induzierten Aggregation $EC_{50}$ |
|---|---|---|---|
| A | 0,004 µM/l | 0,004 µM/l | 0,5 µM/l |
| B | 0,004 µM/l | 0,008 µM/l | 2,2 µM/l |
| C | 0,032 µM/l | 0,012 µM/l | 0,8 µM/l |
| D | 0,090 µM/l | 0,017 µM/l | 1,2 µM/l |

4. Akute Toxizität

Die akute Toxizität der zu untersuchenden Substanzen wurde orientierend an Gruppen von je 10 Mäusen nach oraler Gabe einer Einzeldosis von 250 mg/kg bestimmt (Beobachtungszeit: 14 Tage). Bei dieser Dosis verstarb keines der eingesetzten Tiere.

Aufgrund ihrer pharmakologischen Eigenschaften eignen sich die neuen Verbindungen und deren physiologisch verträgliche Additionssalze zur Behandlung und zur Prophylaxe thrombo-embolischer Erkrankungen wie Coronarinfarkt, Cerebralinfarkt, sogen. transient ischaemic attacks, Amaurosis fugax, zur Prophylaxe der Arteriosklerose und zur Metastasenprophylaxe sowie zur Behandlung der Ischämie, des Asthmas und von Allergien.

Die neuen Verbindungen und deren physiologisch verträglichen Additionssalze sind auch bei der Behandlung von Erkrankungen nützlich, bei denen eine thromboxanvermittelte Konstriktion oder $PGE_2$ vermittelte Dilatation der Kapillaren eine Rolle spielen, z.B. bei der pulmonalen Hypertension. Außerdem können diese zur Verminderung des Schweregrades einer Transplantatabstoßung, zur Verminderung der renalen Toxizität von Substanzen wie Cyclosporin, zur Behandlung von Nierenerkrankungen, insbesondere zur Therapie oder Prophylaxe von Nierenveränderungen im Zusammenhang von Hypertension, systemischem Lupus oder Ureterobstruktionen und bei Schockzuständen im Zusammenhang mit Sepsis, Trauma oder Verbrennungen eingesetzt werden.

Die zur Erzielung einer entsprechenden Wirkung erforderliche Dosierung beträgt zweckmäßigerweise zwei- bis viermal täglich 0,3 bis 4 mg/kg Körpergewicht, vorzugsweise 0,3 bis 2 mg/kg Körpergewicht. Hierzu lassen sich die erfindungsgemäß hergestellten Verbindungen der Formel I, gegebenenfalls in Kombination mit anderen Wirksubstanzen, zusammen mit einem oder mehreren inerten üblichen Trägerstoffen und/oder Verdünnungsmitteln, z.B. mit Maisstärke, Milchzucker, Rohrzucker, mikrokristalliner Zellulose, Magnesiumstearat, Polyvinylpyrrolidon, Zitronensäure, Weinsäure, Wasser, Wasser/Äthanol, Wasser/Glycerin, Wasser/Sorbit, Wasser/Polyäthylenglykol, Propylenglykol,

Cetylstearylalkohol, Carboxymethylcellulose oder fetthaltige Substanzen wie Hartfett oder deren geeignete Gemische, in übliche galenische Zubereitungen wie Tabletten, Dragées, Kapseln, Pulver, Suspensionen oder Zäpfchen einarbeiten.

Ein weiterer Gegenstand der vorliegenden Erfindung sind neue Arzneimittel, enthaltend eine erfindungsgemäß hergestellte Verbindung der Formel I und einen PDE-Hemmer oder ein Lysemittel.

Als PDE-Hemmer kommt hierbei beispielsweise
2,6-Bis(diäthanolamino)-4,8-dipiperidino-pyrimido[5,4-d]pyrimidin (Dipyridamol),
2,6-Bis(diäthanolamino)-4-piperidino-pyrimido[5,4-d]pyrimidin (Mopidamol),
2-(4-Methoxy-phenyl)-5(6)-(5-methyl-3-oxo-4,5-dihydro-2H-6-pyridazinyl)-benzimidazol (Pimobendan),
2-(4-Hydroxy-phenyl)-5(6)-(5-methyl-3-oxo-4,5-dihydro-2H-6-pyridazinyl)-benzimidazol,
1-(1-Oxido-thiomorpholino)-3-piperazino-5-methyl-isochinolin,
6-[4-(3,4-Dichlorphenylsulfinyl)-butoxy]-3,4-dihydrocarbostyril und
6-[4-(2-Pyridylsulfonyl)-butoxy]carbostyril, wobei die perorale Tagesdosis
für Dipyridamol 2,5 bis 7,5 mg/kg, vorzugsweise 5 mg/kg,
für Mopidamol 15 bis 25 mg/kg, vorzugsweise 20 mg/kg,
für 2-(4-Methoxy-phenyl)-5(6)-(5-methyl-3-oxo-4,5-dihydro-2H-6-pyridazinyl)-benzimidazol 0,05 bis 0,15 mg/kg, vorzugsweise 0,08 bis 0,10 mg/kg,
für 2-(4-Hydroxy-phenyl)-5(6)-(5-methyl-3-oxo-4,5-dihydro-2H-6-pyridazinyl)-benzimidazol 0,05 bis 0,15 mg/kg, vorzugsweise 0,08 bis 0,10 mg/kg,
für 1-(1-Oxido-thiomorpholino)-3-piperazino-5-methyl-isochinolin 0,20 bis 2,00 mg/kg, vorzugsweise 0,40 bis 1,00 mg/kg,
für 6-[4-(3,4-Dichlorphenylsulfinyl)-butoxy]-3,4-dihydrocarbostyril 0,10 bis 1,00 mg/kg, vorzugsweise 0,20 bis 0,50 mg/kg und
für 6-[4-(2-Pyridylsulfonyl)-butoxy]carbostyril 0,10 bis 1,00 mg/kg, vorzugsweise 0,20 bis 0,50 mg/kg, beträgt,
und als Lysemittel Plasminogen-Aktivatoren wie t-PA, rt-PA, Streptokinase, Eminase oder Urokinase in Betracht, wobei die Lysemittel parenteral, vorzugsweise jedoch intravenös, verabreicht werden, z.B. t-PA oder rt-PA in einer Dosierung zwischen 15 und 100 mg pro Patient, Urokinase in einer Dosis zwischen 250 000 und 3 000 000 Einheiten pro Patient, Eminase in einer Dosierung von ungefähr 30 mg pro Patient und Streptokinase in einer Dosis zwischen $5 \times 10^4$ bis $3 \times 10^7$ IU jeweils innerhalb 5 Minuten und 24 Stunden.

Zur pharmazeutischen Anwendung läßt sich eine neue Kombination, enthaltend 1 bis 500 mg eines PDE-Hemmers, vorzugsweise jedoch 2 bis 75 mg, plus 10 bis 300 mg einer erfindungsgemäß hergestellten Verbindung der Formel I, vorzugsweise jedoch 10 bis 200 mg, sowie deren physiologisch verträgliche Additionssalze zusammen mit einem oder mehreren inerten üblichen Trägerstoffen und/oder Verdünnungsmitteln, z.B. mit Maisstärke, Milchzucker, Rohrzucker, mikrokristalliner Zellulose, Magnesiumstearat, Polyvinylpyrrolidon, Zitronensäure, Weinsäure, Wasser, Wasser/Äthanol, Wasser/Glycerin, Wasser/Sorbit, Wasser/Polyäthylenglykol, Propylenglykol, Cetylstearylalkohol, Carboxymethylcellulose oder fetthaltige Substanzen wie Hartfett oder deren geeignete Gemische, in übliche galenische Zubereitungen wie Tabletten, Dragées, Kapseln, Pulver, Suspensionen oder Zäpfchen einarbeiten. Hierbei erfolgt die Applikation am Erwachsenen zur Erzielung einer entsprechenden Wirkung 2 bis 4 mal täglich, vorzugsweise jedoch 3 bis 4 mal täglich.

Desweiteren läßt sich zur pharmazeutischen Anwendung eine neue Kombination, enthaltend ein Lysemittel in den vorstehend erwähnten Dosierungen plus 10 bis 300 mg einer erfindungsgemäß hergestellten Verbindung der Formel I, vorzugsweise jedoch 10 bis 200 mg, sowie deren physiologisch verträgliche Additionssalze in die üblichen parenteralen, vorzugsweise in die üblichen intravenösen, Applikationsformen wie Ampullen oder Infusionen einarbeiten, wobei die Applikation innerhalb von 5 Minuten und 24 Stunden erfolgen kann.

Selbstverständlich können die einzelnen Wirksubstanzen der vorstehenden Kombinationen gewünschtenfalls appliziert werden.

Die nachfolgenden Beispiele sollen die Erfindung näher erläutern:

Herstellung der Ausgangsprodukte:

Beispiel I

6-(4-Aminophenyl)-6-(3-pyridyl)-hex-5-ensäuremethylester

a) 4-Acetylaminophenyl-3-pyridyl-keton

980 g Aluminiumtrichlorid werden langsam mit 155 ml Dimethylformamid versetzt. Zu dieser Mischung gibt man bei 90-110°C nacheinander 342 g Nicotinsäurechlorid-hydrochlorid und 206 g N-Acetylanilin. Anschließend wird die Reaktionsmischung mit 600 ml Ethylenchlorid versetzt, danach auf Eis gegeben und unter Kühlung durch Zugabe von 15 N Natronlauge neutralisiert. Die wäßrige Phase wird mit Methylenchlorid extrahiert. Die vereinigten organischen Phasen werden eingeengt und der Rückstand wird aus Methanol umkristallisiert.

Ausbeute: 44 % der Theorie,
Schmelzpunkt: 189-191°C

| $C_{14}H_{12}N_2O_3$ (240,26) | | | | | | |
|---|---|---|---|---|---|---|
| Ber.: | C | 69,99 | H | 5,03 | N | 11,66 |
| Gef.: | | 69,87 | | 5,14 | | 11,58 |

b) 6-(4-Acetylaminophenyl)-6-(3-pyridyl)-hex-5-ensäure

Zu einer Suspension von 307 g 4-Carboxybutyl-triphenylphosphoniumbromid und 233 g Kalium-tert.butylat in 2,8 l Tetrahydrofuran gibt man bei -40°C 140 g 4-Acetylaminophenyl-3-pyridyl-keton und rührt 2 Stunden. Die Reaktionsmischung wird durch Zugabe von Eiswasser zersetzt und eingeengt. Der Rückstand wird in Wasser aufgenommen und mit Essigsäureethylester gewaschen. Die wäßrige Phase wird auf pH 5-6 angesäuert und mit Essigsäureethylester extrahiert. Die organische Phase wird eingeengt und der Rückstand aus Essigsäureethylester/Diisopropylether umkristallisiert.
Ausbeute: 86 % der Theorie,
Schmelzpunkt: 155-156°C

| $C_{19}H_{20}N_2O_3$ (324,38) | | | | | | |
|---|---|---|---|---|---|---|
| Ber.: | C | 70,35 | H | 6,21 | N | 8,64 |
| Gef.: | | 70,19 | | 6,27 | | 8,66 |

c) 6-(4-Aminophenyl)-6-(3-pyridyl)-hex-5-ensäuremethylester

65 g 6-(4-Acetylaminophenyl)-6-(3-pyridyl)-hex-5-ensäure werden in einer Mischung aus 300 ml Methanol und 150 ml gesättigter methanolischer Salzsäure 2 Stunden unter Rückfluß gekocht. Die Reaktionsmischung wird mit 500 ml Wasser versetzt, durch Zugabe von Natriumcarbonat neutralisiert und mit Essigsäureethylester extrahiert. Die organische Phase wird gewaschen, getrocknet und eingeengt.
Ausbeute: 71 % der Theorie,
Öl, $R_f$-Wert: 0,72 (Kieselgel; Methylenchlorid/Aceton = 9:1)

| $C_{18}H_{20}N_2O_2$ (296,37) | | | | | | |
|---|---|---|---|---|---|---|
| Ber.: | C | 72,95 | H | 6,80 | N | 9,45 |
| Gef.: | | 72,83 | | 6,85 | | 9,23 |

Analog Beispiel I wird folgende Verbindung erhalten:
6-(4-Methylaminophenyl)-6-(3-pyridyl)-hex-5-ensäuremethylester
Öl, $R_f$-Wert: 0,56 (Kieselgel; Methylenchlorid/Ethanol = 20:1)

| $C_{19}H_{22}N_2O_2$ (310,40) | | | | | | |
|---|---|---|---|---|---|---|
| Ber.: | C | 73,52 | H | 7,14 | N | 9,03 |
| Gef.: | | 73,35 | | 7,24 | | 8,91 |

Beispiel II

6-(3-Aminophenyl)-6-(3-pyridyl)-hex-5-ensäuremethylester

a) 3-Acetylaminophenyl-3-pyridylketon

114 g 3-Nitrophenyl-3-pyridylketon werden in 1 000 ml Essigsäure und 35 g Raney-Nickel 2 Stunden bei 50°C und 5 bar hydriert. Der Katalysator wird abfiltriert und das Filtrat mit 80 ml Essigsäureanhydrid versetzt. Nach 30 Minuten bei Raumtemperatur wird eingeengt und der Rückstand in Essigsäureethylester aufgenommen. Die organische Phase wird mit wäßriger Kaliumcarbonatlösung gewaschen und über Natriumsulfat getrocknet. Das Lösungsmittel wird abgezogen und der Rückstand aus Essigsäureethylester/Diisopropylether umkristallisiert.
Ausbeute: 69 % der Theorie,
Schmelzpunkt: 116-117°C

| $C_{14}H_{12}N_2O_2$ (240,26) | | | | | | |
|---|---|---|---|---|---|---|
| Ber.: | C | 69,99 | H | 5,03 | N | 11,66 |
| Gef.: | | 70,01 | | 5,11 | | 11,81 |

b) 6-(3-Acetylaminophenyl)-6-(3-pyridyl)-hex-5-ensäure

Zu einer Mischung aus 217 g 4-Carboxybutyl-triphenylphosphoniumbromid und 154 g Kalium-tert.butylat in 1,8 l Tetrahydrofuran gibt man bei -25°C 94 g 3-Acetylaminophenyl-3-pyridylketon. Nach 2 Stunden Rühren bei Raumtemperatur wird die Reaktionsmischung mit 200 ml Wasser versetzt und anschließend weitgehend eingeengt. Der Rückstand wird in 500 ml Wasser aufgenommen und mit Essigsäureethylester gewaschen. Anschließend wird die wäßrige Phase durch Zugabe von Zitronensäure neutralisiert und mit Essigsäureethylester extrahiert. Die organische Phase wird eingeengt und der Rückstand aus Essigsäureethylester/Aceton umkristallisiert.
Ausbeute: 85 % der Theorie,
Schmelzpunkt: 86-89°C

| $C_{19}H_{20}N_2O_3$ (324,38) | | | | | | |
|---|---|---|---|---|---|---|
| Ber.: | C | 70,35 | H | 6,21 | N | 8,64 |
| Gef.: | | 70,15 | | 6,36 | | 8,50 |

c) 6-(3-Aminophenyl)-6-(3-pyridyl)-hex-5-ensäuremethylester

65 g 6-(3-Acetylaminophenyl)-6-(3-pyridyl)-hex-5-ensäure werden in einer Mischung aus 400 ml Methanol und 200 ml methanolischer Salzsäure 4 Stunden unter Rückfluß gekocht. Das Lösungsmittel wird abgezogen und der Rückstand in Wasser aufgenommen. Die wäßrige Phase wird mit Essigsäureethylester gewaschen und durch Zugabe von 4N Natronlauge auf pH 8-9 eingestellt. Die wäßrige Phase wird mit Essigsäureethylester extrahiert. Die organische Phase wird gewaschen, getrocknet und eingeengt.
Ausbeute: 71 % der Theorie,
Öl, $R_f$-Wert: 0,55 (Kieselgel; Methylenchlorid/Ethanol = 9:1)

| $C_{18}H_{20}N_2O_2$ (296,37) | | | | | | |
|---|---|---|---|---|---|---|
| Ber.: | C | 72,95 | H | 6,80 | N | 9,45 |
| Gef.: | | 72,83 | | 6,91 | | 9,18 |

Analog Beispiel II werden folgende Verbindungen erhalten:
5-(3-Aminophenyl)-5-(3-pyridyl)-pent-4-ensäuremethylester Harz, $R_f$-Wert: 0,58 (Kieselgel; Methylenchlorid/Ethanol =

20:1)

| $C_{17}H_{18}N_2O_2$ (282,34) | | | | | | |
|---|---|---|---|---|---|---|
| Ber.: | C | 72,32 | H | 6,43 | N | 9,92 |
| Gef.: | | 72,29 | | 6,55 | | 9,70 |

7-(3-Aminophenyl)-7-(3-pyridyl)-hept-6-ensäuremethylester
Harz, $R_f$-Wert: 0,63 (Kieselgel; Methylenchlorid/Ethanol = 20:1)

| $C_{19}H_{22}N_2O_2$ (310,40) | | | | | | |
|---|---|---|---|---|---|---|
| Ber.: | C | 73,52 | H | 7,14 | N | 9,03 |
| Gef.: | | 73,41 | | 7,18 | | 8,89 |

Beispiel III

6-(3-Methylaminophenyl)-6-(3-pyridyl)-hex-5-ensäuremethylester

a) N-Acetyl-3-methylaminophenyl-3-pyridylketon

Zu 84 g 3-Acetylaminophenyl-3-pyridylketon in 600 ml Dimethylformamid gibt man portionsweise unter Kühlung 17 g Natriumhydrid und anschließend 22 ml Methyljodid. Man rührt eine Stunde bei Raumtemperatur und zersetzt die Reaktionsmischung durch Zugabe von 100 ml Wasser. Das Lösungsmittel wird abgezogen und der Rückstand in Essigsäureethylester aufgenommen. Die organische Phase wird gewaschen, getrocknet und eingeengt. Der Rückstand wird über eine Kieselgelsäule mit Methylenchlorid/Ethanol (30:1) gereinigt.
Öl, $R_f$-Wert: 0,45 (Kieselgel; Methylenchlorid/Ethanol = 30:1)

| $C_{15}H_{14}N_2O_2$ (254,29) | | | | | | |
|---|---|---|---|---|---|---|
| Ber.: | C | 70,85 | H | 5,55 | N | 11,02 |
| Gef.: | | 70,96 | | 5,65 | | 10,92 |

b) 6-(3-Methylaminophenyl)-6-(3-pyridyl)-hex-5-ensäuremethylester

Hergestellt aus N-Acetyl-3-methylaminophenyl-3-pyridylketon und 4-Carboxybutyl-triphenylphosphoniumbromid analog Beispiel IIb und anschließende Veresterung analog Beispiel IIc.
Öl, $R_f$-Wert: 0,56 (Kieselgel; Methylenchlorid/Ethanol = 20:1)

| $C_{19}H_{22}N_2O_2$ (310,40) | | | | | | |
|---|---|---|---|---|---|---|
| Ber.: | C | 73,52 | H | 7,14 | N | 9,03 |
| Gef.: | | 73,53 | | 7,20 | | 8,84 |

Beispiel IV

(+)-E-2-(4-Chlorphenyl)-cyclopropan-1-carbonsäure

a) E/Z-2-(4-Chlorphenyl)-cyclopropan-1-carbonsäureethylester

Eine Mischung von 85 g Diazoessigsäureethylester und 100 g 4-Chlorstyrol werden innerhalb von 2 Stunden zu 350 ml siedendem Xylol getropft. Man erhitzt eine weitere Stunde auf 120°C und zieht das Lösungsmittel im Vakuum ab. Der Rückstand wird im Hochvakuum fraktioniert.
Ausbeute: 43 % der Theorie,
Siedepunkt: 105-112°C (0,05 Torr)

| $C_{12}H_{13}Cl0_2$ (224,69) | | | | |
|---|---|---|---|---|
| Ber.: | C | 64,15 | H | 5,83 |
| Gef.: | | 64,33 | | 6,03 |

b) E-2-(4-Chlorphenyl)-cyclopropan-1-carbonsäure und Z-2-(4-Chlorphenyl)-cyclopropan-1-carbonsäure

Zu einer siedenden Mischung aus 69 g E/Z-2-(4-Chlorphenyl)-cyclopropan-1-carbonsäureethylester und 8,8 g Natriumhydroxid in 180 ml Ethanol und 50 ml Wasser tropft man innerhalb von 5 Stunden 160 ml Wasser und destilliert gleichzeitig 150 ml Ethanol ab. Anschließend wird mit Essigsäureeethylester der Z-2-(4-Chlorphenyl)-cyclopropan-1-carbonsäureethylester aus der Reaktionsmischung extrahiert. Die wäßrige Phase wird sauer gestellt und mit Essigsäureethylester extrahiert. Die organische Phase wird eingeengt und der Rückstand aus Petrolether umkristallisiert, wobei man 20 g E-2-(4-Chlorphenyl)-cyclopropan-1-carbonsäure erhält.
Die esterhaltige organische Phase wird eingeengt und der Rückstand in einer Mischung aus 8,8 g Natriumhydroxid, 180 ml Ethanol und 50 ml Wasser zum Sieden erhitzt. Zur siedenden Reaktionsmischung tropft man innerhalb von 5 Stunden 160 ml Wasser zu und destilliert gleichzeitig 150 ml Ethanol ab. Anschließend wird die Reaktionslösung angesäuert und mit Essigsäureethylester extrahiert. Die organische Phase wird eingeengt und der Rückstand aus Benzin/Essigsäureethylester umkristallisiert, wobei man 10,1 g Z-2-(4-Chlorphenyl)-cyclopropan-1-carbonsäure erhält.
Schmelzpunkt (E-Isomer): 116-117°C
Schmelzpunkt (Z-Isomer): 128-129°C

| $C_{10}H_{19}Cl0_2$ (196,63) | | | | |
|---|---|---|---|---|
| Ber.: | C | 61,08 | H | 4,61 |
| Gef. (E-Isomer): | | 60,96 | | 4,66 |
| Gef. (Z-Isomer): | | 61,02 | | 4,76 |

c) (+)- und (-)-E-2-(4-Chlorphenyl)-cyclopropan-1-carbonsäure L-phenyl-glycinolamid

Zu einer Mischung aus 1,96 g E-2-(4-Chlorphenyl)-cyclopropan-1-carbonsäure und 1,01 g N-Methylmorpholin in 30 ml Tetrahydrofuran werden bei -55°C 1,35 g Chlorameisensäureisobutylester zugetropft. Nach 10 Minuten gibt man 1,37 g L-Phenylglycinol zu und läßt auf Raumtemperatur erwärmen. Nach 2 Stunden wird die Reaktionsmischung mit 30 ml Wasser versetzt, eingeengt und der entstandene Niederschlag abgesaugt. Das erhaltene Produkt wird durch Säulenchromatographie an Kieselgel mit Methylenchlorid/Aceton (15:1) in die Diastereomeren getrennt. Die reinen Fraktionen werden anschließend aus Essigsäureethylester umkristallisiert.

Ausbeute: 30 % der Theorie an Diastereomer A und
26 % der Theorie an Diastereomer B

| $C_{18}H_{18}ClNO_2$ (315,80) | | | | | | |
|---|---|---|---|---|---|---|
| Ber.: | C | 68,46 | H | 5,74 | N | 4,44 |
| Gef. (Diastereomer A): | | 68,59 | | 5,86 | | 4,57 |
| Gef. (Diastereomer B): | | 68,27 | | 5,81 | | 4,54 |

d) (+)-E-2-(4-Chlorphenyl)-cyclopropan-1-carbonsäure

19 g Diastereomer A werden in 200 ml Dioxan und 200 ml 4N Salzsäure gelöst und 4 Stunden am Rückfluß gekocht. Die Reaktionsmischung wird eingeengt, der entstandene Niederschlag abgesaugt und aus Petrolether/Essigsäureethylester umkristallisiert.
Ausbeute: 91 % der Theorie,
Schmelzpunkt: 114-115°C
Drehwert: $[\alpha]_D^{20}$ = +351° (c = 1,2; Chloroform)

| $C_{10}H_9ClO_2$ (196,63) | | | | |
|---|---|---|---|---|
| Ber.: | C | 61,08 | H | 4,61 |
| Gef.: | | 61,12 | | 4,64 |

Analog Beispiel IV werden folgende Verbindungen erhalten:
(-)-E-2-(4-Chlorphenyl)-cyclopropan-1-carbonsäure
Schmelzpunkt: 114-115°C
Drehwert: $[\alpha]_D^{20}$ = -348° (c = 1,2; Chloroform)

| $C_{10}H_9ClO_2$ (196,63) | | | | |
|---|---|---|---|---|
| Ber.: | C | 61,08 | H | 4,61 |
| Gef.: | | 61,12 | | 4,55 |

(+)-Z-2-(4-Chlorphenyl)-cyclopropan-1-carbonsäure
Schmelzpunkt: 95-96°C
Drehwert: $[\alpha]_D^{20}$ = +2,6° (c = 1,2; Chloroform)

| $C_{10}H_9ClO_2$ (196,63) | | | | |
|---|---|---|---|---|
| Ber.: | C | 61,08 | H | 4,61 |
| Gef.: | | 61,09 | | 4,64 |

(-)-Z-2-(4-Chlorphenyl)-cyclopropan-1-carbonsäure

Schmelzpunkt: 95-96°C
Drehwert: $[\alpha]_D^{20}$ = -2,6° (c = 1,2; Chloroform)

| $C_{10}H_9ClO_2$ (196,63) | | | | |
|---|---|---|---|---|
| Ber.: | C | 61,08 | H | 4,61 |
| Gef.: | | 61,24 | | 4,64 |

Herstellung der Endprodukte:

Beispiel 1

6-[4-(4-Chlorbenzoylamino)-phenyl]-6-(3-pyridyl)-hex-5-ensäure

Zu einer Mischung aus 3 g 6-(4-Aminophenyl)-6-(3-pyridyl)-hex-5-ensäure-methylester und 2,1 g 4-Chlorbenzoesäurechlorid tropft man bei 5-10°C 5 ml Triethylamin. Nach 30 Minuten wird die Reaktionsmischung mit Wasser gewaschen und eingeengt. Der Rückstand wird mit 30 ml Ethanol und 5 ml 10N Natronlauge versetzt und 30 Minuten auf 50°C erwärmt. Die Reaktionsmischung wird eingeengt und der Rückstand in Wasser aufgenommen. Die wäßrige Phase wird mit Essigsäureethylester gewaschen, durch Zugabe von Zitronensäure neutralisiert und anschließend mit Essigsäureethylester extrahiert. Die organische Phase wird mit Wasser gewaschen, getrocknet und eingeengt und der Rückstand aus Essigsäureethylester/Diisopropylether umkristallisiert.
Ausbeute: 64 % der Theorie,
Schmelzpunkt: 94-95°C

| $C_{24}H_{21}ClN_2O_3$ (420,90) | | | | | | |
|---|---|---|---|---|---|---|
| Ber.: | C | 68,49 | H | 5,03 | N | 6,66 |
| Gef.: | | 68,21 | | 5,13 | | 6,60 |

Analog Beispiel 1 werden folgende neue Verbindungen erhalten:
6-(4-Phenylacetylaminophenyl)-6-(3-pyridyl)-hex-5-ensäure
Schmelzpunkt: 168-169°C

| $C_{25}H_{24}N_2O_3$ (400,48) | | | | | | |
|---|---|---|---|---|---|---|
| Ber.: | C | 74,98 | H | 6,03 | N | 6,99 |
| Gef.: | | 74,85 | | 6,25 | | 7,02 |

6-[4-(2-Phenylbenzoylamino)-phenyl]-6-(3-pyridyl)-hex-5-ensäure
Schmelzpunkt: 182-183°C

| $C_{30}H_{26}N_2O_3$ (462,55) | | | | | | |
|---|---|---|---|---|---|---|
| Ber.: | C | 77,90 | H | 5,67 | N | 6,06 |
| Gef.: | | 77,80 | | 5,89 | | 6,18 |

6-[4-(1-(4-Chlorphenyl)-cyclopropylcarboxamido)-phenyl]-6-(3-pyridyl)-hex-5-ensäure

Schmelzpunkt: 147-148°C

| $C_{27}H_{25}ClN_2O_3$ (460,96) | | | | | | |
|---|---|---|---|---|---|---|
| Ber.: | C | 70,35 | H | 5,47 | N | 6,08 |
| Gef.: | | 70,54 | | 5,46 | | 6,06 |

6-[4-(E-m-Chlorcinnamoylamino)-phenyl]-6-(3-pyridyl)-hex-5-ensäure
Schmelzpunkt: 101-102°C

| $C_{26}H_{23}ClN_2O_3$ (446,93) | | | | | | |
|---|---|---|---|---|---|---|
| Ber.: | C | 69,87 | H | 5,19 | N | 6,27 |
| Gef.: | | 69,79 | | 5,37 | | 6,53 |

6-[4-(3-(4-Chlorphenyl)-propionylamino)-phenyl]-6-(3-pyridyl)-hex-5-ensäure
Schmelzpunkt: 148°C

| $C_{26}H_{25}ClN_2O_3$ (448,95) | | | | | | |
|---|---|---|---|---|---|---|
| Ber.: | C | 69,56 | H | 5,61 | N | 6,24 |
| Gef.: | | 69,45 | | 5,62 | | 6,41 |

6-[4-(4-Chlorphenylacetylamino)-phenyl]-6-(3-pyridyl)-hex-5-ensäure
Schmelzpunkt: 176-177°C

| $C_{25}H_{23}ClN_2O_3$ | | | | | | |
|---|---|---|---|---|---|---|
| Ber.: | C | 69,04 | H | 5,33 | N | 6,44 |
| Gef.: | | 68,93 | | 5,51 | | 6,31 |

6-[4-(4-Phenylbutanoylamino)-phenyl]-6-(3-pyridyl)-hex-5-ensäure
Schmelzpunkt: 144-145°C

| $C_{27}H_{28}N_2O_3$ (428,53) | | | | | | |
|---|---|---|---|---|---|---|
| Ber.: | C | 75,68 | H | 6,59 | N | 6,54 |
| Gef.: | | 75,64 | | 6,72 | | 6,45 |

6-[4-(3-Phenylpropinoylamino)-phenyl]-6-(3-pyridyl)-hex-5-ensäure
Schmelzpunkt: 95-96°C

| $C_{26}H_{22}N_2O_3$ (410,47) | | | | | | |
|---|---|---|---|---|---|---|
| Ber.: | C | 76,08 | H | 5,40 | N | 6,83 |
| Gef.: | | 75,85 | | 5,60 | | 6,95 |

6-[4-(E-2-Phenylcyclopropyl-1-carboxamido)-phenyl]-6-(3-pyridyl)-hex-5-ensäure
Schmelzpunkt: 175-176°C

| $C_{27}H_{26}N_2O_3$ (426,51) | | | | | | |
|---|---|---|---|---|---|---|
| Ber.: | C | 76,09 | H | 6,14 | N | 6,56 |
| Gef.: | | 76,06 | | 6,18 | | 6,48 |

(+)-5E-6-[4-(E-2-(4-Chlorphenyl)-cyclopropyl-1-carboxamido)-phenyl]-6-(3-pyridyl)-hex-5-ensäure
Schmelzpunkt: 167-168°C
Drehwert: $[\alpha]_D^{20}$ = +292° (c = 1,2; Methanol)

| $C_{27}H_{25}ClN_2O_3$ (460,96) | | | | | | |
|---|---|---|---|---|---|---|
| Ber.: | C | 70,35 | H | 5,47 | N | 6,07 |
| Gef.: | | 70,18 | | 5,56 | | 6,11 |

(-)-5E-6-[4-(E-2-(4-Chlorphenyl)-cyclopropyl-1-carboxamido)-phenyl]-6-(3-pyridyl)-hex-5-ensäure
Schmelzpunkt: 167-168°C
Drehwert: $[\alpha]_D^{20}$ = -294° (c = 1,2; Methanol)

| $C_{27}H_{25}ClN_2O_3$ (460,96) | | | | | | |
|---|---|---|---|---|---|---|
| Ber.: | C | 70,35 | H | 5,47 | N | 6,07 |
| Gef.: | | 70,28 | | 5,56 | | 6,00 |

(+)-5E-6-[4-(Z-2-(4-Chlorphenyl)-cyclopropyl-1-carboxamido)-phenyl]-6-(3-pyridyl)-hex-5-ensäure
Schmelzpunkt: 110-111°C
Drehwert: $[\alpha]_D^{20}$ = +10,5° (c = 1,2; Methanol)

| $C_{27}H_{25}ClN_2O_3$ (460,96) | | | | | | |
|---|---|---|---|---|---|---|
| Ber.: | C | 70,35 | H | 5,47 | N | 6,07 |
| Gef.: | | 70,24 | | 5,62 | | 6,22 |

(-)-5E-6-[4-(Z-2-(4-Chlorphenyl)-cyclopropyl-1-carboxamido)-phenyl]-6-(3-pyridyl)-hex-5-ensäure
Schmelzpunkt: 110-111°C
Drehwert: $[\alpha]_D^{20}$ = -11° (c = 1,2; Methanol)

| $C_{27}H_{25}ClN_2O_3$ (460,96) | | | | | | |
|---|---|---|---|---|---|---|
| Ber.: | C | 70,35 | H | 5,47 | N | 6,07 |
| Gef.: | | 70,20 | | 5,55 | | 6,07 |

6-[4-(Z-2-(4-Bromphenyl)-cyclopropyl-1-carboxamido)-phenyl]-6-(3-pyridyl)-hex-5-ensäure

Schmelzpunkt: 204-205°C

| $C_{27}H_{25}BrN_{2}0_{3}$ (505,41) | | | | | | |
|---|---|---|---|---|---|---|
| Ber.: | C | 64,17 | H | 4,99 | N | 5,54 |
| Gef.: | | 64,00 | | 5,01 | | 5,63 |

6-[4-(Z-2-(4-Chlorbenzoyl)-cyclopropyl-1-carboxamido)-phenyl]-6-(3-pyridyl)-hex-5-ensäure
Schmelzpunkt: 193-194°C

| $C_{28}H_{25}ClN_{2}0_{4}$ (488,96) | | | | | | |
|---|---|---|---|---|---|---|
| Ber.: | C | 68,78 | H | 5,15 | N | 5,72 |
| Gef.: | | 68,68 | | 5,23 | | 5,73 |

6-[4-(N-Methyl-3-(4-Chlorphenyl)-propionylamino)-phenyl]-6-(3-pyridyl)-hex-5-ensäure
Schmelzpunkt: 87-90°C

| $C_{27}H_{27}ClN_{2}0_{3}$ (462,97) | | | | | | |
|---|---|---|---|---|---|---|
| Ber.: | C | 70,05 | H | 5,88 | N | 6,05 |
| Gef.: | | 69,95 | | 5,87 | | 5,95 |

6-[4-(N-Methyl-Z-2-(4-chlorphenyl)-cyclopropyl-1-carboxamido)-phenyl]-6-(3-pyridyl)-hex-5-ensäure
Schaum, $R_f$-Wert: 0,41 (Kieselgel; Methylenchlorid/Ethanol = 20:1)

| $C_{28}H_{27}ClN_{2}0_{3}$ (474,99) | | | | | | |
|---|---|---|---|---|---|---|
| Ber.: | C | 70,80 | H | 5,73 | N | 5,90 |
| Gef.: | | 70,71 | | 5,75 | | 5,66 |

6-[4-(N-Methyl-E-2-(4-chlorphenyl)-cyclopropyl-1-carboxamido)-phenyl]-6-(3-pyridyl)-hex-5-ensäure
Schmelzpunkt: 148-150°C

| $C_{28}H_{27}ClN_{2}0_{3}$ (474,99) | | | | | | |
|---|---|---|---|---|---|---|
| Ber.: | C | 70,80 | H | 5,73 | N | 5,90 |
| Gef.: | | 70,60 | | 5,79 | | 5,80 |

6-[3-(2-Phenylbenzoylamino)-phenyl]-6-(3-pyridyl)-hex-5-ensäure
Schmelzpunkt: 214-216°C

| $C_{30}H_{26}N_{2}0_{3}$ (462,55) | | | | | | |
|---|---|---|---|---|---|---|
| Ber.: | C | 77,90 | H | 5,67 | N | 6,06 |
| Gef.: | | 77,79 | | 5,86 | | 5,97 |

6-[3-(E-p-Chlorcinnamoylamino)-phenyl]-6-(3-pyridyl)-hex-5-ensäure
Schmelzpunkt: 173-174°C

| $C_{26}H_{23}ClN_{2}0_{3}$ (446,93) | | | | | | |
|---|---|---|---|---|---|---|
| Ber.: | C | 69,87 | H | 5,19 | N | 6,27 |
| Gef.: | | 69,68 | | 5,27 | | 6,05 |

6-[3-(4-Chlorbenzoylamino)-phenyl]-6-(3-pyridyl)-hex-5-ensäure
Schmelzpunkt: 186°C

| $C_{24}H_{21}ClN_{2}0_{3}$ (420,90) | | | | | | |
|---|---|---|---|---|---|---|
| Ber.: | C | 68,49 | H | 5,03 | N | 6,66 |
| Gef.: | | 68,29 | | 5,14 | | 6,55 |

6-[3-(3-Chlorbenzoylamino)-phenyl]-6-(3-pyridyl)-hex-5-ensäure
Schmelzpunkt: 173°C

| $C_{24}H_{21}ClN_{2}0_{3}$ (420,90) | | | | | | |
|---|---|---|---|---|---|---|
| Ber.: | C | 68,49 | H | 5,03 | N | 6,66 |
| Gef.: | | 68,33 | | 5,13 | | 6,73 |

6-[3-(E-o-Chlorcinnamoylamino)-phenyl]-6-(3-pyridyl)-hex-5-ensäure
Schmelzpunkt: 199-200°C

| $C_{26}H_{23}ClN_{2}0_{3}$ (446,93) | | | | | | |
|---|---|---|---|---|---|---|
| Ber.: | C | 69,87 | H | 5,19 | N | 6,27 |
| Gef.: | | 69,67 | | 5,15 | | 6,29 |

6-[3-(E-m-Chlorcinnamoylamino)-phenyl]-6-(3-pyridyl)-hex-5-ensäure
Schmelzpunkt: 187-188°C

| $C_{26}H_{23}ClN_{2}0_{3}$ (446,93) | | | | | | |
|---|---|---|---|---|---|---|
| Ber.: | C | 69,87 | H | 5,19 | N | 6,27 |
| Gef.: | | 69,67 | | 5,29 | | 6,26 |

6-[3-(2-Naphthoylamino)-phenyl]-6-(3-pyridyl)-hex-5-ensäure
Schmelzpunkt: 207-208°C

| $C_{27}H_{24}N_{2}0_{3}$ (424,50) | | | | | | |
|---|---|---|---|---|---|---|
| Ber.: | C | 76,39 | H | 5,70 | N | 6,60 |
| Gef.: | | 76,53 | | 5,64 | | 6,47 |

6-[3-(4-Methoxybenzoylamino)-phenyl]-6-(3-pyridyl)-hex-5-ensäure
Schmelzpunkt: 168-170°C

| $C_{25}H_{24}H_{2}O_{4}$ (416,48) | | | | | | |
|---|---|---|---|---|---|---|
| Ber.: | C | 72,10 | H | 5,81 | N | 6,73 |
| Gef.: | | 71,99 | | 5,83 | | 6,80 |

6-[3-(3-Phenylpropionylamino)-phenyl]-6-(3-pyridyl)-hex-5-ensäure
Schmelzpunkt: 157°C

| $C_{26}H_{26}N_{2}O_{3}$ (414,50) | | | | | | |
|---|---|---|---|---|---|---|
| Ber.: | C | 75,33 | H | 6,32 | N | 6,76 |
| Gef.: | | 75,34 | | 6,35 | | 6,91 |

6-[3-(4-Phenylbenzoylamino)-phenyl]-6-(3-pyridyl)-hex-5-ensäure
Schmelzpunkt: 163-164°C

| $C_{30}H_{26}N_{2}O_{3}$ (462,55) | | | | | | |
|---|---|---|---|---|---|---|
| Ber.: | C | 77,90 | H | 5,67 | N | 6,06 |
| Gef.: | | 77,83 | | 5,50 | | 6,13 |

(+)-5E-6-[3-(E-2-(4-Chlorphenyl)-cyclopropyl-1-carboxamido)-phenyl]-6-(3-pyridyl)-hex-5-ensäure
Schaum, $R_f$-Wert: 0,33 (Kieselgel; Methylenchlorid/Ethanol = 20:1)
Drehwert: $[\alpha]_D^{20}$ = +242° (c = 1,2; Methanol)

| $C_{27}H_{25}ClN_{2}O_{3}$ (460,96) | | | | | | |
|---|---|---|---|---|---|---|
| Ber.: | C | 70,35 | H | 5,47 | N | 6,07 |
| Gef.: | | 70,28 | | 5,70 | | 5,83 |

(-)-5E-6-[3-(E-2-(4-Chlorphenyl)-cyclopropyl-1-carboxamido)-phenyl]-6-(3-pyridyl)-hex-5-ensäure
Schaum, $R_f$-Wert: 0,33 (Kieselgel; Methylenchlorid/Ethanol = 20:1)
Drehwert: $[\alpha]_D^{20}$ = -243° (c = 1,2; Methanol)

| $C_{27}H_{25}ClN_{2}O_{3}$ (460,96) | | | | | | |
|---|---|---|---|---|---|---|
| Ber.: | C | 70,35 | H | 5,47 | N | 6,07 |
| Gef.: | | 70,20 | | 5,67 | | 5,95 |

(+)-5E-6-[3-(Z-2-(4-Chlorphenyl)-cyclopropyl-1-carboxamido)-phenyl]-6-(3-pyridyl)-hex-5-ensäure
Schmelzpunkt: 221-222°C

Drehwert: $[\alpha]_D^{20}$ = +87° (c = 1,2; Dimethylformamid)

| $C_{27}H_{25}ClN_2O_3$ (460,96) | | | | | | |
|---|---|---|---|---|---|---|
| Ber.: | C | 70,35 | H | 5,47 | N | 6,07 |
| Gef.: | | 70,38 | | 5,53 | | 5,96 |

(-)-5E-6-[3-(Z-2-(4-Chlorphenyl)-cyclopropyl-1-carboxamido)-phenyl]-6-(3-pyridyl)-hex-5-ensäure
Schmelzpunkt: 220-221°C
Drehwert: $[\alpha]_D^{20}$ = -87° (c = 1,2; Dimethylformamid)

| $C_{27}H_{25}ClN_2O_3$ (460,96) | | | | | | |
|---|---|---|---|---|---|---|
| Ber.: | C | 70,35 | H | 5,47 | N | 6,07 |
| Gef.: | | 70,21 | | 5,48 | | 6,07 |

6-[3-(E-2-(4-Bromphenyl)-cyclopropyl-1-carboxamido)-phenyl]-6-(3-pyridyl)-hex-5-ensäure
Schmelzpunkt: 165-168°C

| $C_{27}H_{25}BrN_2O_3$ (505,41) | | | | | | |
|---|---|---|---|---|---|---|
| Ber.: | C | 64,17 | H | 4,99 | N | 5,54 |
| Gef.: | | 64,00 | | 5,10 | | 5,69 |

6-[3-(1-(4-Chlorphenyl)-cyclopropylcarboxamido)-phenyl]-6-(3-pyridyl)-hex-5-ensäure
Schmelzpunkt: 149-150°C

| $C_{27}H_{25}ClN_2O_3$ (460,96) | | | | | | |
|---|---|---|---|---|---|---|
| Ber.: | C | 70,35 | H | 5,47 | N | 6,07 |
| Gef.: | | 70,19 | | 5,59 | | 6,11 |

6-[3-(3-(4-Chlorphenyl)-propionylamino)-phenyl]-6-(3-pyridyl)-hex-5-ensäure
Schmelzpunkt: 163°C

| $C_{26}H_{25}ClN_2O_3$ (448,95) | | | | | | |
|---|---|---|---|---|---|---|
| Ber.: | C | 69,56 | H | 5,61 | N | 6,24 |
| Gef.: | | 69,56 | | 5,64 | | 6,40 |

6-[3-(4-Chlorphenylacetylamino)-phenyl]-6-(3-pyridyl)-hex-5-ensäure
Schmelzpunkt: 192-193°C

| $C_{25}H_{23}ClN_2O_3$ (434,92) | | | | | | |
|---|---|---|---|---|---|---|
| Ber.: | C | 69,04 | H | 5,33 | N | 6,44 |
| Gef.: | | 68,87 | | 5,37 | | 6,48 |

6-[3-(4-Phenylbutanoylamino)-phenyl]-6-(3-pyridyl)-hex-5-ensäure
Schmelzpunkt: 116-117°C

| $C_{27}H_{28}N_{2}0_{3}$ (428,53) | | | | | | |
|---|---|---|---|---|---|---|
| Ber.: | C | 75,68 | H | 5,59 | N | 6,54 |
| Gef.: | | 75,69 | | 5,70 | | 6,42 |

6-[3-(4-Phenylpropinoylamino)-phenyl]-6-(3-pyridyl)-hex-5-ensäure
Schmelzpunkt: 159-160°C

| $C_{26}H_{22}N_{2}0_{3}$ (410,47) | | | | | | |
|---|---|---|---|---|---|---|
| Ber.: | C | 76,08 | H | 5,40 | N | 6,83 |
| Gef.: | | 75,96 | | 5,50 | | 7,03 |

6-[3-(Z-2-(4-Bromphenyl)-cyclopropyl-1-carboxamido)-phenyl-6-(3-pyridyl)-hex-5-ensäure
Schmelzpunkt: 204-205°C

| $C_{27}H_{25}BrN_{2}0_{3}$ (505,41) | | | | | | |
|---|---|---|---|---|---|---|
| Ber.: | C | 64,17 | H | 4,99 | N | 5,54 |
| Gef.: | | 64,00 | | 5,01 | | 5,63 |

6-[3-(3,3-Diphenylpropionylamino)-phenyl]-6-(3-pyridyl)-hex-5-ensäure
Schaum, $R_f$-Wert: 0,30 (Kieselgel; Methylenchlorid/Ethanol = 20:1)

| $C_{32}H_{30}N_{2}0_{3}$ (490,60) | | | | | | |
|---|---|---|---|---|---|---|
| Ber.: | C | 78,34 | H | 6,16 | N | 5,71 |
| Gef.: | | 78,21 | | 6,29 | | 5,67 |

6-[3-(E-3,3-Dichlor-2-(4-chlorphenyl)-cyclopropyl-1-carboxamido)-phenyl]-6-(3-pyridyl)-hex-5-ensäure
Schmelzpunkt: 65°C

| $C_{27}H_{23}Cl_{3}N_{2}0_{3}$ (529,85) | | | | | | |
|---|---|---|---|---|---|---|
| Ber.: | C | 61,21 | H | 4,38 | N | 5,29 |
| Gef.: | | 61,04 | | 4,54 | | 5,05 |

6-[3-(N-Methyl-E-o-chlorcinnamoylamino)-phenyl]-6-(3-pyridyl)-hex-5-ensäure
Schmelzpunkt: 128-130°C

| $C_{27}H_{25}ClN_{2}0_{3}$ (460,96) | | | | | | |
|---|---|---|---|---|---|---|
| Ber.: | C | 70,35 | H | 5,47 | N | 6,08 |
| Gef.: | | 70,32 | | 5,43 | | 6,10 |

6-[3-(N-Methyl-3-(4-chlorphenyl)-propionylamino)-phenyl]-6-(3-pyridyl)-hex-5-ensäure
Öl, $R_f$-Wert: 0,73 (Kieselgel; Methylenchlorid/Ethanol = 9:1)

| $C_{27}H_{27}ClN_2O_3$ (462,98) | | | | | | |
|---|---|---|---|---|---|---|
| Ber.: | C | 70,05 | H | 5,88 | N | 6,05 |
| Gef.: | | 69,88 | | 5,99 | | 5,88 |

6-[3-(N-Methyl-3-(2-chlorphenyl)-propionylamino)-phenyl]-6-(3-pyridyl)-hex-5-ensäure
Schmelzpunkt: 122-124°C

| $C_{27}H_{27}ClN_2O_3$ (462,98) | | | | | | |
|---|---|---|---|---|---|---|
| Ber.: | C | 70,05 | H | 5,88 | N | 6,05 |
| Gef.: | | 69,85 | | 5,73 | | 6,04 |

6-[3-(N-Methyl-3,3-diphenylpropionylamino)-phenyl]-6-(3-pyridyl)-hex-5-ensäure
Schaum, $R_f$-Wert: 0,46 (Kieselgel; Methylenchlorid/Ethanol = 20:1)

| $C_{33}H_{32}N_2O_3$ (504,63) | | | | | | |
|---|---|---|---|---|---|---|
| Ber.: | C | 78,55 | H | 6,39 | N | 5,55 |
| Gef.: | | 78,43 | | 6,49 | | 5,48 |

6-[3-(N-Methyl-3-(4-methoxyphenyl)-propionylamino)-phenyl]-6-(3-pyridyl)-hex-5-ensäure
Schmelzpunkt: 135°C

| $C_{28}H_{30}N_2O_4$ (458,56) | | | | | | |
|---|---|---|---|---|---|---|
| Ber.: | C | 73,34 | H | 6,59 | N | 6,11 |
| Gef.: | | 73,20 | | 6,70 | | 6,17 |

6-[3-(N-Methyl-3-phenylpropinoylamino)-phenyl]-6-(3-pyridyl)-hex-5-ensäure
Schmelzpunkt: 123-125°C

| $C_{27}H_{24}N_2O_3$ (424,50) | | | | | | |
|---|---|---|---|---|---|---|
| Ber.: | C | 76,40 | H | 5,70 | N | 6,60 |
| Gef.: | | 76,30 | | 5,73 | | 6,88 |

7-[3-(E-2-(4-Chlorphenyl)-cyclopropyl-1-carboxamido)-phenyl]-7-(3-pyridyl)-hept-6-ensäure
Schaum, $R_f$-Wert: 0,15 (Kieselgel; Methylenchlorid/Aceton = 9:1)

| $C_{28}H_{27}ClN_2O_3$ (474,99) | | | | | | |
|---|---|---|---|---|---|---|
| Ber.: | C | 70,80 | H | 5,73 | N | 5,90 |
| Gef.: | | 70,64 | | 5,87 | | 6,00 |

7-[3-(3-(4-Chlorphenyl)-propionylamino)-phenyl]-7-(3-pyridyl)-hept-6-ensäure
Schaum, $R_f$-Wert: 0,20 (Kieselgel; Essigsäureethylester)

| $C_{27}H_{27}ClN_2O_3$ (462,98) | | | | | | |
|---|---|---|---|---|---|---|
| Ber.: | C | 70,05 | H | 5,88 | N | 6,05 |
| Gef.: | | 69,90 | | 5,90 | | 6,06 |

5-[3-(E-2-(4-Chlorphenyl)-cyclopropyl-1-carboxamido)-phenyl]-5-(3-pyridyl)-pent-4-ensäure
Schaum, $R_f$-Wert: 0,22 (Kieselgel; Methylenchlorid/Ethanol = 19:1)

| $C_{26}H_{25}ClN_2O_3$ (448,95) | | | | | | |
|---|---|---|---|---|---|---|
| Ber.: | C | 69,56 | H | 5,61 | N | 6,24 |
| Gef.: | | 69,37 | | 5,35 | | 6,22 |

5-[3-(3-(4-Chlorphenyl)-propionylamino)-phenyl]-5-(3-pyridyl)-pent-4-ensäure
Schmelzpunkt: 200-201°C

| $C_{25}H_{23}ClN_2O_3$ (434,92) | | | | | | |
|---|---|---|---|---|---|---|
| Ber.: | C | 69,04 | H | 5,33 | N | 6,44 |
| Gef.: | | 68,86 | | 5,39 | | 6,42 |

Beispiel 2

6-[3-(4-Chlorphenoxyacetylamino)-phenyl]-6-(3-pyridyl)-hex-5-ensäure

2,25 g 6-(3-Aminophenyl)-6-(3-pyridyl)-hex-5-ensäuremethylester werden in 30 ml Methylenchlorid gelöst. Zu dieser Lösung gibt man bei 0°C nacheinander 1,65 g 4-Chlorphenoxyessigsäurechlorid und 2,5 ml Triethylamin und rührt 2 Stunden bei Raumtemperatur. Die Reaktionsmischung wird mit Wasser gewaschen und danach eingeengt. Der Rückstand wird in einer Mischung aus 20 ml Ethanol und 16 ml 0,5N Natronlauge gelöst und 24 Stunden bei Raumtemperatur gerührt. Die Reaktionsmischung wird bei Raumtemperatur eingeengt, mit Wasser versetzt und mit Essigsäureethylester gewaschen. Die wäßrige Phase wird durch Zugabe von Zitronensäure neutralisiert und mit Essigsäureethylester extrahiert. Die organische Phase wird mit Wasser gewaschen, getrocknet, eingeengt und der Rückstand aus Essigsäureethylester/Diisopropylether umkristallisiert.
Ausbeute: 49 % der Theorie,
Schmelzpunkt: 178-179°C

| $C_{25}H_{23}ClN_2O_4$ (450,92) | | | | | | |
|---|---|---|---|---|---|---|
| Ber.: | C | 66,59 | H | 5,14 | N | 6,21 |
| Gef.: | | 66,48 | | 5,29 | | 5,99 |

Analog Beispiel 2 werden folgende Verbindungen erhalten:
6-[3-(2-(4-Chlorphenoxy)-isobutyroylamino)-phenyl]-6-(3-pyridyl)-hex-5-ensäure

Schmelzpunkt: 92°C

| $C_{27}H_{27}ClN_2O_4$ (478,98) | | | | | | |
|---|---|---|---|---|---|---|
| Ber.: | C | 67,71 | H | 5,68 | N | 5,85 |
| Gef.: | | 67,56 | | 5,79 | | 5,67 |

6-[3-(2-Chlorphenoxyacetylamino)-phenyl]-6-(3-pyridyl)-hex-5-ensäure
Schmelzpunkt: 134-136°C

| $C_{25}H_{23}ClN_2O_4$ (450,92) | | | | | | |
|---|---|---|---|---|---|---|
| Ber.: | C | 66,59 | H | 5,14 | N | 6,21 |
| Gef.: | | 66,51 | | 5,13 | | 6,16 |

6-[3-(2,4-Dichlorphenoxyacetylamino)-phenyl]-6-(3-pyridyl)-hex-5-ensäure
Schmelzpunkt: 148-150°C

| $C_{25}H_{22}Cl_2N_2O_4$ (485,37) | | | | | | |
|---|---|---|---|---|---|---|
| Ber.: | C | 61,87 | H | 4,57 | N 5,77 | |
| Gef.: | | 61,74 | | 4,45 | | 5,81 |

6-[3-(N-Methyl-2-chlorphenoxyacetylamino)-phenyl]-6-(3-pyridyl)-hex-5-ensäure
Schmelzpunkt: 118-119°C

| $C_{26}H_{25}ClN_2O_4$ (464,95) | | | | | | |
|---|---|---|---|---|---|---|
| Ber.: | C | 67,17 | H | 5,42 | N | 6,02 |
| Gef.: | | 66,99 | | 5,46 | | 5,99 |

6-[3-(N-Methyl-(2-chlorphenoxy)-isobutyroylamino)-phenyl]-6-(3-pyridyl)-hex-5-ensäure
Schaum, $R_f$-Wert: 0,45 (Kieselgel; Methylenchlorid/Ethanol = 20:1)

| $C_{28}H_{29}ClN_2O_4$ (493,00) | | | | | | |
|---|---|---|---|---|---|---|
| Ber.: | C | 68,22 | H | 5,93 | N | 5,68 |
| Gef.: | | 68,10 | | 6,09 | | 5,65 |

6-[3-(N-Methyl-4-chlorphenoxyacetylamino)-phenyl]-6-(3-pyridyl)-hex-5-ensäure
Schmelzpunkt: 128-132°C

| $C_{26}H_{25}ClN_2O_4$ (464,95) | | | | | | |
|---|---|---|---|---|---|---|
| Ber.: | C | 67,17 | H | 5,42 | N | 6,02 |
| Gef.: | | 67,26 | | 5,32 | | 6,13 |

6-[3-(N-Methyl-2,4-dichlorphenoxyacetylamino)-phenyl]-6-(3-pyridyl)-hex-5-ensäure
Schmelzpunkt: 146°C

| $C_{26}H_{24}Cl_2N_2O_4$ (499,40) | | | | | | |
|---|---|---|---|---|---|---|
| Ber.: | C | 62,53 | H | 4,84 | N | 5,61 |
| Gef.: | | 62,47 | | 4,90 | | 5,40 |

Beispiel 3

6-[3-(N-Methyl-3-(3-indolyl)-propionylamino)-phenyl]-6-(3-pyridyl)-hex-5-ensäure

Eine Mischung aus 1,9 g 3-(3-Indolyl)-propionsäure, 2,3 g 6-(3-Methylaminophenyl)-6-(3-pyridyl)-hex-5-ensäure-methylester und 1,8 g Carbonyldiimidazol werden in 50 ml Tetrahydrofuran 48 Stunden unter Rückfluß gekocht. Die Reaktionsmischung wird eingeengt, der Rückstand in Wasser aufgenommen und mit Essigsäureethylester extrahiert. Die organische Phase wird eingeengt und der Rückstand in einer Mischung aus 20 ml Ethanol und 6 ml 4N Natronlauge 30 Minuten bei 50°C verseift. Die Reaktionsmischung wird durch Zugabe von Zitronensäure neutralisiert und mit Essigsäureethylester extrahiert. Die organische Phase wird eingeengt und der Rückstand aus Essigsäureethylester/Diisopropylether umkristallisiert.
Ausbeute: 25 % der Theorie,
Schmelzpunkt: 142-144°C

| $C_{29}H_{29}N_3O_3$ (467,57) | | | | | | |
|---|---|---|---|---|---|---|
| Ber.: | C | 74,50 | H | 6,25 | N | 8,99 |
| Gef.: | | 74,32 | | 6,23 | | 8,85 |

Beispiel 4

6-[3-(3-(4-Chlorphenyl)-ureido)-phenyl]-6-(3-pyridyl)-hex-5-ensäure

2,4 g 6-(3-Aminophenyl)-6-(3-pyridyl)-hex-5-ensäuremethylester und 1,5 g 4-Chlorphenylisocyanat werden in 50 ml Tetrahydrofuran eine Stunde bei Raumtemperatur gerührt. Die Reaktionsmischung wird eingeengt, der Rückstand mit Wasser versetzt und mit Essigsäureethylester/Ethanol (9:1) extrahiert. Die organische Phase wird eingeengt und der Rückstand in 60 ml Ethanol und 6 ml 4N Natronlauge eine Stunde bei 50°C verseift. Die Reaktionsmischung wird eingengt, der Rückstand wird in 100 ml Wasser aufgenommen und mit Essigsäureethylester extrahiert. Die wäßrige Phase wird durch Zugabe von Zitronensäure neutralisiert, wobei das Produkt ausfällt. Der Niederschlag wird abgesaugt, mit Wasser und Diisopropylether gewaschen und aus Essigsäureethylester/Diisopropylether umkristallisiert.
Ausbeute: 52 % der Theorie,
Schmelzpunkt: 172-173°C

| $C_{24}H_{22}ClN_3O_3$ (435,91) | | | | | | |
|---|---|---|---|---|---|---|
| Ber.: | C | 66,13 | H | 5,09 | N | 9,64 |
| Gef.: | | 66,04 | | 5,22 | | 9,71 |

Analog Beispiel 4 werden folgende Verbindungen erhalten:
6-[3-(3-(3-Chlorphenyl)-ureido)-phenyl]-6-(3-pyridyl)-hex-5-ensäure

Schmelzpunkt: 151-152°C

| $C_{24}H_{22}ClN_3O_3$ (435,91) | | | | | | |
|---|---|---|---|---|---|---|
| Ber.: | C | 66,13 | H | 5,09 | N | 9,64 |
| Gef.: | | 66,05 | | 5,18 | | 9,52 |

6-[3-(3-(2,5-Dimethylphenyl)-ureido)-phenyl]-6-(3-pyridyl)-hex-5-ensäure
Schmelzpunkt: 137°C (Zers.),

| $C_{26}H_{27}N_3O_3$ (429,52) | | | | | | |
|---|---|---|---|---|---|---|
| Ber.: | C | 72,71 | H | 6,34 | N | 9,78 |
| Gef.: | | 72,58 | | 6,35 | | 9,75 |

6-[3-(3-(2-Chlorphenyl)-ureido)-phenyl]-6-(3-pyridyl)-hex-5-ensäure
Schmelzpunkt: 170-172°C

| $C_{24}H_{22}ClN_3O_3$ (435,91) | | | | | | |
|---|---|---|---|---|---|---|
| Ber.: | C | 66,13 | H | 5,09 | N | 9,64 |
| Gef.: | | 66,03 | | 5,22 | | 9,45 |

6-[3-(3-(1-Naphthyl)-ureido)-phenyl]-6-(3-pyridyl)-hex-5-ensäure
Schmelzpunkt: 173°C

| $C_{28}H_{25}N_3O_3$ (451,53) | | | | | | |
|---|---|---|---|---|---|---|
| Ber.: | C | 74,48 | H | 5,58 | N | 9,31 |
| Gef.: | | 74,32 | | 5,53 | | 9,16 |

6-[3-(3-(3,4-Dichlorphenyl)-ureido)-phenyl]-6-(3-pyridyl)-hex-5-ensäure
Schmelzpunkt: 178°C

| $C_{24}H_{21}Cl_2N_3O_4$ (470,36) | | | | | | |
|---|---|---|---|---|---|---|
| Ber.: | C | 61,29 | H | 4,50 | N | 8,93 |
| Gef.: | | 61,24 | | 4,61 | | 9,10 |

6-[3-(3-(2,4-Dichlorphenyl)-ureido)-phenyl]-6-(3-pyridyl)-hex-5-ensäure
Schmelzpunkt: 126°C

| $C_{24}H_{21}Cl_2N_3O_3$ (470,36) | | | | | | |
|---|---|---|---|---|---|---|
| Ber.: | C | 61,29 | H | 4,50 | N | 8,93 |
| Gef.: | | 61,01 | | 4,69 | | 8,99 |

6-[3-(3-(2,3-Dichlorphenyl)-ureido)-phenyl]-6-(3-pyridyl)-hex-5-ensäure
Schmelzpunkt: 196°C

| $C_{24}H_{21}Cl_2N_3O_3$ (470,36) | | | |
|---|---|---|---|
| Ber.: | C 61,29 | H 4,50 | N 8,93 |
| Gef.: | 61,14 | 4,66 | 8,82 |

6-[3-(3-(3-Nitrophenyl)-ureido)-phenyl]-6-(3-pyridyl)-hex-5-ensäure
Schmelzpunkt: 207-208°C

| $C_{24}H_{22}N_4O_5$ (446,47) | | | | | | |
|---|---|---|---|---|---|---|
| Ber.: | C | 64,57 | H | 4,97 | N | 12,55 |
| Gef.: | | 64,58 | | 4,97 | | 12,36 |

6-[3-(3-(4-Carboxyphenyl)-ureido)-phenyl]-6-(3-pyridyl)-hex-5-ensäure
Schmelzpunkt: ab 118°C (Zers.),

| $C_{25}H_{23}N_3O_5$ (445,48) | | | | | | |
|---|---|---|---|---|---|---|
| Ber.: | C | 67,41 | H | 5,20 | N | 9,43 |
| Gef.: | | 67,27 | | 5,17 | | 9,21 |

6-[3-(3-Benzylureido)-phenyl]-6-(3-pyridyl)-hex-5-ensäure
Schmelzpunkt: 166-168°C

| $C_{25}H_{25}N_3O_3$ (415,49) | | | | | | |
|---|---|---|---|---|---|---|
| Ber.: | C | 72,27 | H | 6,06 | N | 10,11 |
| Gef.: | | 72,10 | | 6,07 | | 9,91 |

6-[3-(3-(4-tert.Butylphenyl)-ureido)-phenyl]-6-(3-pyridyl)-hex-5-ensäure
Schmelzpunkt: 180-182°C

| $C_{28}H_{31}N_3O_3$ (457,57) | | | | | | |
|---|---|---|---|---|---|---|
| Ber.: | C | 73,50 | H | 6,83 | N | 9,18 |
| Gef.: | | 73,38 | | 6,78 | | 9,20 |

6-[3-(3-Benzoylureido)-phenyl]-6-(3-pyridyl)-hex-5-ensäure
Schmelzpunkt: 190-191°C

| $C_{25}H_{23}N_3O_4$ (429,48) | | | | | | |
|---|---|---|---|---|---|---|
| Ber.: | C | 69,92 | H | 5,40 | N | 9,78 |
| Gef.: | | 69,72 | | 5,34 | | 9,58 |

6-[3-(3-Cyclohexylureido)-phenyl]-6-(3-pyridyl)-hex-5-ensäure
Schmelzpunkt: 163-164°C

| $C_{24}H_{29}N_3O_3$ (407,51) | | | | | | |
|---|---|---|---|---|---|---|
| Ber.: | C | 70,74 | H | 7,17 | N | 10,31 |
| Gef.: | | 70,66 | | 7,36 | | 10,38 |

6-[3-(3-(tert.-Butyl)-ureido)-phenyl]-6-(3-pyridyl)-hex-5-ensäure
Umsetzung in einer Mischung aus Tetrahydrofuran, Dimethylformamid und 4-Dimethylaminopyridin unter Erwärmen.
Schmelzpunkt: 165°C

| $C_{22}H_{27}N_3O_3$ (381,48) | | | | | | |
|---|---|---|---|---|---|---|
| Ber.: | C | 69,27 | H | 7,13 | N | 11,02 |
| Gef.: | | 69,24 | | 7,05 | | 10,95 |

6-[3-(3-(2,4-Dichlorphenyl)-1-methylureido)-phenyl]-6-(3-pyridyl)-hex-5-ensäure
Schmelzpunkt: 139-141°C

| $C_{25}H_{23}Cl_2N_3O_3$ (484,38) | | | | | | |
|---|---|---|---|---|---|---|
| Ber.: | C | 61,99 | H | 4,79 | N | 8,68 |
| Gef.: | | 61,86 | | 4,91 | | 8,58 |

6-[3-(3-(4-Carboxyphenyl)-1-methylureido)-phenyl]-6-(3-pyridyl)-hex-5-ensäure
Reinigung durch Säulenchromatographie an Kieselgel mit Methylenchlorid/Ethanol (30:1)
Schaum, $R_f$-Wert: 0,52 (Kieselgel; Methylenchlorid/Ethanol = 20:1)

| $C_{26}H_{25}N_3O_5$ (459,50) | | | | | | |
|---|---|---|---|---|---|---|
| Ber.: | C | 67,96 | H | 5,48 | N | 9,14 |
| Gef.: | | 67,88 | | 5,56 | | 8,96 |

6-[4-(3-(3-Chlorphenyl)-ureido)-phenyl]-6-(3-pyridyl)-hex-5-ensäure
Schmelzpunkt: 175-176°C

| $C_{24}H_{22}ClN_3O_3$ (435,91) | | | | | | |
|---|---|---|---|---|---|---|
| Ber.: | C | 66,13 | H | 5,09 | N | 9,64 |
| Gef.: | | 65,99 | | 5,14 | | 9,58 |

6-[4-(3-(2,4-Dichlorphenyl)-1-methylureido)-phenyl]-6-(3-pyridyl)-hex-5-ensäure

Schmelzpunkt: 150-152°C

| $C_{25}H_{23}Cl_2N_3O_3$ (484,38) | | | | | | |
|---|---|---|---|---|---|---|
| Ber.: | C | 61,99 | H | 4,79 | N | 8,67 |
| Gef.: | | 61,88 | | 4,78 | | 8,49 |

5-[3-(3-(3-Chlorphenyl)-ureido)-phenyl]-5-(3-pyridyl)-pent-4-ensäure
Reinigung durch Säulenchromatographie an Kieselgel mit Methylenchlorid/Ethanol (9:1)
Schaum, $R_f$-Wert: 0,25 (Kieselgel; Methylenchlorid/Ethanol = 19:1)

| $C_{23}H_{20}ClN_3O_3$ (421,88) | | | | | | |
|---|---|---|---|---|---|---|
| Ber.: | C | 65,48 | H | 4,78 | N | 9,96 |
| Gef.: | | 65,23 | | 4,87 | | 9,66 |

7-[3-(3-(3-Chlorphenyl)-ureido)-phenyl]-7-(3-pyridyl)-hept-6-ensäure
Schmelzpunkt: 140-141°C

| $C_{25}H_{24}ClN_3O_3$ (449,94) | | | | | | |
|---|---|---|---|---|---|---|
| Ber.: | C | 66,74 | H | 5,38 | N | 9,34 |
| Gef.: | | 66,63 | | 5,34 | | 9,25 |

Beispiel 5

6-[4-(3,3-Diphenylureido)-phenyl]-6-(3-pyridyl)-hex-5-ensäure

Eine Mischung aus 2,4 g 6-(4-Aminophenyl)-6-(3-pyridyl)-hex-5-ensäure-methylester und 2,1 g N,N-Diphenylcarbamoylchlorid wird in 25 ml Pyridin 18 Stunden bei Raumtemperatur gerührt. Das Lösungsmittel wird abgezogen und der Rückstand in Essigsäureethylester aufgenommen. Die organische Phase wird mit Wasser gewaschen, getrocknet und eingeengt. Der Rückstand wird in einer Mischung aus 20 ml Ethanol und 6 ml 4N Natronlauge 30 Minuten bei 50°C verseift. Die Reaktionslösung wird mit Wasser verdünnt und mit Essigsäureethylester extrahiert. Anschließend wird die wäßrige Phase durch Zugabe von Zitronensäure neutralisiert, der entstandene Niederschlag abgesaugt und aus Essigsäureethylester/Isopropanol umkristallisiert.
Ausbeute: 55 % der Theorie,
Schmelzpunkt: 155-156°C

| $C_{30}H_{27}N_3O_3$ (477,56) | | | | | | |
|---|---|---|---|---|---|---|
| Ber.: | C | 75,45 | H | 5,70 | N | 8,80 |
| Gef.: | | 75,28 | | 5,89 | | 8,68 |

Analog Beispiel 5 werden folgende Verbindungen erhalten:
6-[4-(3-Methyl-3-phenylureido)-phenyl]-6-(3-pyridyl)-hex-5-ensäure

Schmelzpunkt: 118-120°C

| $C_{25}H_{25}N_3O_3$ (415,29) | | | | | | |
|---|---|---|---|---|---|---|
| Ber.: | C | 72,27 | H | 6,07 | N | 10,11 |
| Gef.: | | 72,19 | | 6,16 | | 10,00 |

6-[4-(1,3-Dimethyl-3-phenylureido)-phenyl]-6-(3-pyridyl)-hex-5-ensäure
Harz, $R_f$-Wert: 0,60 (Kieselgel; Methylenchlorid/Ethanol = 20:1)

| $C_{26}H_{27}N_3O_3$ (429,52) | | | | | | |
|---|---|---|---|---|---|---|
| Ber.: | C | 72,71 | H | 6,34 | N | 9,78 |
| Gef.: | | 72,55 | | 6,44 | | 9,54 |

6-[3-(1,3-Dimethyl-3-phenylureido)-phenyl]-6-(3-pyridyl)-hex-5-ensäure
Harz, $R_f$-Wert: 0,40 (Kieselgel; Methylenchlorid/Ethanol = 20:1)

| $C_{26}H_{27}N_3O_3$ (429,52) | | | | | | |
|---|---|---|---|---|---|---|
| Ber.: | C | 72,71 | H | 6,34 | N | 9,78 |
| Gef.: | | 72,53 | | 6,34 | | 9,59 |

Beispiel 6

6-[3-(3-(4-Chlorphenyl)-thioureido)-phenyl]-6-(3-pyridyl)-hex-5-ensäure

2,4 g 6-(3-Aminophenyl)-6-(3-pyridyl)-hex-5-ensäuremethylester und 1,7 g 4-Chlorphenylisothiocyanat werden in 30 ml Tetrahydrofuran 1,5 Stunden bei Raumtemperatur gerührt. Das Lösungsmittel wird abgezogen, der Rückstand in Wasser aufgenommen und mit Essigsäureethylester extrahiert. Die organische Phase wird eingeengt und der Rückstand in einer Mischung aus 20 ml Ethanol und 6 ml 4N Natronlauge 90 Minuten bei Raumtemperatur verseift. Die Reaktionsmischung wird mit Wasser verdünnt, mit Essigsäureethylester extrahiert und anschließend durch Zugabe von Zitronensäure neutralisiert. Die wäßrige Phase wird mit Essigsäureethylester extrahiert. Die organische Phase wird eingeengt und der Rückstand über eine Kieselgelsäule mit Methylenchlorid/Ethanol (20:1) gereinigt.
Ausbeute: 64 % der Theorie,
Schaum, $R_f$-Wert: 0,31 (Kieselgel; Methylenchlorid/Aceton = 2:1)

| $C_{24}H_{22}ClN_3O_2S$ (451,98) | | | | | | |
|---|---|---|---|---|---|---|
| Ber.: | C | 63,78 | H | 4,91 | N | 9,30 |
| Gef.: | | 63,62 | | 5,03 | | 9,11 |

Analog Beispiel 6 werden folgende Verbindungen erhalten:
6-[3-(3-(3-Chlorphenyl)-thioureido)-phenyl]-6-(3-pyridyl)-hex-5-ensäure

Schmelzpunkt: 142-144°C (Essigsäureethylester/tert.Butylmethylether)

| $C_{24}H_{22}ClN_3O_2S$ (451,98) | | | | | | |
|---|---|---|---|---|---|---|
| Ber.: | C | 63,78 | H | 4,91 | N | 9,30 |
| Gef.: | | 63,69 | | 5,11 | | 9,10 |

6-[3-(3-(2-Chlorphenyl)-thioureido)-phenyl]-6-(3-pyridyl)-hex-5-ensäure
Schmelzpunkt: 146-148°C (Isopropanol/Diisopropylether)

| $C_{24}H_{22}ClN_3O_2S$ (451,98) | | | | | | |
|---|---|---|---|---|---|---|
| Ber.: | C | 63,78 | H | 4,91 | N | 9,30 |
| Gef.: | | 63,87 | | 5,06 | | 9,10 |

6-[3-(3-(2,4-Dichlorphenyl)-thioureido)-phenyl]-6-(3-pyridyl)-hex-5-ensäure
Schmelzpunkt: 154-155°C (Essigsäureethylester/Diisopropylether)

| $C_{24}H_{21}Cl_2N_3O_2S$ (486,41) | | | | | | |
|---|---|---|---|---|---|---|
| Ber.: | C | 59,26 | H | 4,35 | N | 8,64 |
| Gef.: | | 59,18 | | 4,53 | | 8,37 |

6-[3-(3-(4-Methylphenyl)-thioureido)-phenyl]-6-(3-pyridyl)-hex-5-ensäure
Schmelzpunkt: 137°C (Essigsäureethylester/tert.Butylmethylether)

| $C_{25}H_{25}N_3O_2S$ (431,56) | | | | | | |
|---|---|---|---|---|---|---|
| Ber.: | C | 69,58 | H | 5,84 | N | 9,74 |
| Gef.: | | 69,38 | | 5,92 | | 9,59 |

6-[3-(3-(4-Nitrophenyl)-thioureido)-phenyl]-6-(3-pyridyl)-hex-5-ensäure
Schmelzpunkt: 139°C (Isopropanol/Dioxan)

| $C_{24}H_{22}N_4O_4S$ (462,53) | | | | | | |
|---|---|---|---|---|---|---|
| Ber.: | C | 62,23 | H | 4,79 | N | 12,11 |
| Gef.: | | 62,08 | | 4,88 | | 12,00 |

6-[3-(3-(tert.-Butyl)-thioureido)-phenyl]-6-(3-pyridyl)-hex-5-ensäure
Umsetzung in einer Mischung aus Tetrahydrofuran, Dimethylformamid und 4-Dimethylaminopyridin unter Erwärmen.
Schmelzpunkt: 137-139°C

| $C_{22}H_{27}N_3O_2S$ (397,54) | | | | | | |
|---|---|---|---|---|---|---|
| Ber.: | C | 66,47 | H | 6,85 | N | 10,57 |
| Gef.: | | 66,49 | | 6,95 | | 10,48 |

6-[3-(3-(2,4-Dichlorphenyl)-1-methylthioureido)-phenyl]-6-(3-pyridyl)-hex-5-ensäure
Schmelzpunkt: 124-126°C (Essigsäureethylester/Diisopropylether)

| $C_{25}H_{23}Cl_2N_3O_2S$ (500,44) | | | | | | |
|---|---|---|---|---|---|---|
| Ber.: | C | 60,00 | H | 4,63 | N | 8,40 |
| Gef.: | | 59,95 | | 4,82 | | 8,25 |

6-[4-(3-(2,4-Dichlorphenyl)-1-methylthioureido)-phenyl]-6-(3-pyridyl)-hex-5-ensäure
Schmelzpunkt: 127-129°C (Essigsäureethylester/Petrolether)

| $C_{25}H_{23}Cl_2N_3O_2S$ (500,44) | | | | | | |
|---|---|---|---|---|---|---|
| Ber.: | C | 60,00 | H | 4,63 | N | 8,40 |
| Gef.: | | 59,87 | | 4,50 | | 8,56 |

Beispiel 7

6-[3-(N-Methyl-2-hydroxy-2-phenylacetylamino)-phenyl]-6-(3-pyridyl)-hex-5-ensäure

4,66 g 6-(3-Methylaminophenyl)-6-(3-pyridyl)-hex-5-ensäuremethylester, 3,2 g DL-O-Acetylmandelsäurechlorid und 3,5 ml Triethylamin werden in 50 ml Methylenchlorid eine Stunde bei Raumtemperatur gerührt. Die Reaktionsmischung wird mit Wasser gewaschen und eingeengt. Der Rückstand in einer Mischung aus 30 ml Ethanol und 10 ml 4N Natronlauge 30 Minuten bei 50°C verseift. Die Reaktionsmischung wird durch Zugabe von Zitronensäure neutralisiert und mit Essigsäureethylester extrahiert. Die organische Phase wird mit Wasser gewaschen, eingeengt und der Rückstand über eine Kieselgelsäule mit Essigsäureethylester gereinigt.
Ausbeute: 69 % der Theorie,
Schaum, $R_f$-Wert: 0,38 (Kieselgel; Methylenchlorid/Aceton = 20:1)

| $C_{26}H_{26}N_2O_4$ (430,50) | | | | | | |
|---|---|---|---|---|---|---|
| Ber.: | C | 72,54 | H | 6,09 | N | 6,51 |
| Gef.: | | 72,34 | | 6,09 | | 6,52 |

Beispiel 8

Tabletten mit 100 mg (-)-5E-6-[4-(Z-2-(4-Chlorphenyl)-cyclopropyl-1-carboxamido)-phenyl]-6-(3-pyridyl)-hex-5-ensäure

Zusammensetzung:

| 1 Tablette enthält: | |
|---|---|
| Wirkstoff | 100,0 mg |
| Milchzucker | 80,0 mg |
| Maisstärke | 34,0 mg |
| Polyvinylpyrrolidon | 4,0 mg |
| Magnesiumstearat | 2,0 mg |
| | 220,0 mg |

Herstellungsverfahren:

Wirkstoff, Milchzucker und Stärke werden gemischt und mit einer wäßrigen Lösung des Polyvinylpyrrolidons gleichmäßig befeuchtet. Nach Siebung der feuchten Massen (2,0 mm-Maschenweite) und Trocknen im Hordentrockenschrank bei 50°C wird erneut gesiebt (1,5 mm-Maschenweite) und das Schmiermittel zugemischt. Die preßfertige Mischung wird zu Tabletten verarbeitet.

| | |
|---|---|
| Tablettengewicht: | 220 mg |
| Durchmesser: | 9 mm, biplan mit beidseitiger Facette und einseitiger Teilkerbe. |

Beispiel 9

Hartgelatine-Kapseln mit 150 mg (-)-5E-6-[4-(Z-2-(4-Chlorphenyl)-cyclopropyl-1-carboxamido)-phenyl]-6-(3-pyridyl)-hex-5-ensäure

| 1 Kapsel enthält: | |
|---|---|
| Wirkstoff | 150,0 mg |
| Maisstärke getr. ca. | 180,0 mg |
| Milchzucker pulv. ca. | 87,0 mg |
| Magnesiumstearat ca. | 3,0 mg |
| | 320,0 mg |

Herstellung:

Der Wirkstoff wird mit den Hilfsstoffen vermengt, durch ein Sieb von 0,75 mm-Maschenweite gegeben und in einem geeigneten Gerät homogen gemischt. Die Endmischung wird in Hartgelatine-Kapseln der Größe 1 abgefüllt.

| | |
|---|---|
| Kapselfüllung: | ca. 320 mg |
| Kapselhülle: | Hartgelatine-Kapsel Größe 1. |

Beispiel 10

Suppositorien mit 150 mg (-)-5E-6-[4-(Z-2-(4-Chlorphenyl)-cyclopropyl-1-carboxamido)-phenyl]-6-(3-pyridyl)-hex-5-ensäure

| 1 Zäpfchen enthält: | |
|---|---|
| Wirkstoff | 150,0 mg |
| Polyäthylenglykol (M.G. 1500) | 550,0 mg |
| Polyäthylenglykol (M.G. 6000) | 460,0 mg |
| Polyoxyäthylensorbitanmonostearat | 840,0 mg |
| | 2 000,0 mg |

Herstellung:

Nach dem Aufschmelzen der Suppositorienmassen wird der Wirkstoff darin homogen verteilt und die Schmelze in vorgekühlte Formen gegossen.

Beispiel 11

Suspensionen mit 50 mg (-)-5E-6-[4-(Z-2-(4-Chlorphenyl)-cyclopropyl-1-carboxamido)-phenyl]-6-(3-pyridyl)-hex-5-ensäure

| 100 ml Suspension enthalten: | |
|---|---|
| Wirkstoff | 1,0 g |
| Carboxymethylcellulose-Na-Salz | 0,2 g |
| p-Hydroxybenzoesäuremethylester | 0,05 g |
| p-Hydroxybenzoesäurepropylester | 0,01 g |
| Glycerin | 5,0 g |
| Sorbitlösung 70%ig | 50,0 g |
| Aroma | 0,3 g |
| Wasser dest. ad | 100 ml |

Herstellung:

Dest. Wasser wird auf 70°C erhitzt. Hierin wird unter Rühren p-Hydroxybenzoesäuremethylester und -propylester sowie Glycerin und Carboxymethylcellulose-Natriumsalz gelöst. Es wird auf Raumtemperatur abgekühlt und unter Rühren der Wirkstoff zugegeben und homogen dispergiert. Nach Zugabe der Sorbitlösung und des Aromas wird die Suspension zur Entlüftung unter Rühren evakuiert.

5 ml Suspension enthalten 50 mg Wirkstoff.

Beispiel 12

Tabletten mit 150 mg (-)-5E-6-[4-(Z-2-(4-Chlorphenyl)-cyclopropyl-1-carboxamido)-phenyl]-6-(3-pyridyl)-hex-5-ensäure

Zusammensetzung:

| 1 Tablette enthält: | |
|---|---|
| Wirksubstanz | 150,0 mg |
| Milchzucker pulv. | 89,0 mg |
| Maisstärke | 40,0 mg |
| Kolloidale Kieselsäure | 10,0 mg |
| Polyvinylpyrrolidon | 10,0 mg |
| Magnesiumstearat | 1,0 mg |
| | 300,0 mg |

Herstellung:

Die mit Milchzucker, Maisstärke und Kieselsäure gemischte Wirksubstanz wird mit einer 20%igen wäßrigen Polyvinylpyrrolidonlösung befeuchtet und durch ein Sieb mit 1,5 mm-Maschenweite geschlagen.

Das bei 45°C getrocknete Granulat wird nochmals durch dasselbe Sieb gerieben und mit der angegebenen Menge Magnesiumstearat gemischt. Aus der Mischung werden Tabletten gepreßt.

| | |
|---|---|
| Tablettengewicht: | 300 mg |
| Stempel: | 10 mm, flach |

Beispiel 13

Filmtabletten mit 75 mg (-)-5E-6-[4-(Z-2-(4-Chlorphenyl)-cyclopropyl-1-carboxamido)-phenyl]-6-(3-pyridyl)-hex-5-ensäure

| 1 Tablettenkern enthält: | |
|---|---|
| Wirksubstanz | 75,0 mg |
| Calciumphosphat | 93,0 mg |
| Maisstärke | 35,5 mg |
| Polyvinylpyrrolidon | 10,0 mg |
| Hydroxypropylmethylcellulose | 15,0 mg |
| Magnesiumstearat | 1,5 mg |
| | 230,0 mg |

Herstellung:

Die Wirksubstanz wird mit Calciumphosphat, Maisstärke, Polyvinylpyrrolidon, Hydroxypropylmethylcellulose und der Hälfte der angegebenen Menge Magnesiumstearat gemischt. Auf einer Tablettiermaschine werden Preßlinge mit einem Durchmesser von ca. 13 mm hergestellt, diese werden auf einer geeigneten Maschine durch ein Sieb mit 1,5 mm Maschenweite gerieben und mit der restlichen Menge Magnesiumstearat vermischt. Dieses Granulat wird auf einer Tablettiermaschine zu Tabletten mit der gewünschten Form gepreßt.

| | |
|---|---|
| Kerngewicht: | 230 mg |
| Stempel: | 9 mm, gewölbt |

Die so hergestellten Tablettenkerne werden mit einem Film überzogen, der im wesentlichen aus Hydroxypropylmethylcellulose besteht. Die fertigen Filmtabletten werden mit Bienenwachs geglänzt.

| | |
|---|---|
| Filmtablettengewicht: | 245 mg |

Selbstverständlich können alle übrigen Verbindungen der allgemeinen Formel I als Wirkstoffe in den vorstehenden galenischen Zubereitungen eingesetzt werden.

Beispiel 14

Filmtabletten, enthaltend 75 mg (-)-5E-6-[4-(Z-2-(4-Chlorphenyl)-cyclopropyl-1-carboxamido)-phenyl]-6-(3-pyridyl)-hex-5-ensäure (Substanz B) + 75 mg PDE-Hemmer

Eine Pulvermischung aus

| | |
|---|---|
| Dipyridamol | 25 % |
| Substanz B | 25 % |
| Fumarsäure | 15 % |
| Cellulose | 20 % |
| Maisstärke | 8 % |
| Polyvinylpyrrolidon | 6 % |

wird in einem Mischgerät mit Wasser befeuchtet und durch ein Sieb mit der Maschenweite 1.5 mm granuliert. Nach Trocknung und erneuter Siebung mischt man 1 % Magnesiumstearat zu und stellt 10 mm bikonvexe Tabletten von 300 mg her. Diese Tabletten werden solange mit Hydroxypropylmethylcelluloselack besprüht bis sie 312 mg wiegen.

Beispiel 15

Hartgelatinekapseln, enthaltend 200 mg (-)-5E-6-[4-(Z-2-(4-Chlorphenyl)-cyclopropyl-1-carboxamido)-phenyl]-6-(3-pyridyl)-hex-5-ensäure (Substanz B) + 50 mg PDE-Hemmer

10 kg Dipyridamol, 20 kg Fumarsäure, 11,5 kg Polyvinylpyrrolidon, 40 kg Substanz B, 1,5 kg Siliciumdioxid und 0,8 kg Magnesiumstearat mischt man 15 Minuten in einem Kubusmischer. Diese Mischung gibt man über einen Walzenkompaktor, dem ein Trockengranuliergerät mit Siebeinrichtung nachgeschaltet ist. Verwendung findet die Fraktion 0.25-1,0 mm. Die Kapselfüllmaschine wird so eingestellt, daß jede Kapsel der Größe 0 die 50 mg PDE-Hemmer und 200 mg (Substanz B) entsprechende Menge Granulat enthält.

Beispiel 16

Hartgelatinekapseln enthaltend 100 mg (-)-5E-6-[4-(Z-2-(4-Chlorphenyl)-cyclopropyl-1-carboxamido)-phenyl]-6-(3-pyridyl)-hex-5-ensäure (Substanz B) + 250 mg PDE-Hemmer

a) Granulat

125 kg Mopidamol, 50 kg Fumarsäure, 13,5 kg Milchzucker werden gemischt und mit einer Lösung aus Wasser/Polyäthylenglykol 6000 befeuchtet. Nach Granulierung durch ein Sieb mit der Maschenweite 1,0 mm und Trocknung bei 45°C mischt man 1,4 kg Stearinsäure zu.

b) Dragée

100 kg Substanz B, 7,5 kg Hydroxypropylmethylcellulose, 2,5 kg Siliciumdioxid und 15 kg Carboxymethylcellulose werden mit Äthanol befeuchtet und durch ein Sieb mit der Maschenweite 1,5 mm granuliert. Nach Trocknung mischt man 1 kg Magnesiumstearat zu und verpreßt das Granulat zu 126 mg schweren bikonvexen Tabletten mit einem Durchmesser von 5,5 mm.

Diese Kerne überzieht man in mehreren Schritten mit einer Dragiersuspension, bestehend aus 5,6 kg Saccharose, 0,5 kg Gummi-arabicum und 3,8 kg Talcum, bis die Tabletten ein Gewicht von 135 mg haben.

c) Abfüllung

Auf einer Spezial-Kapselmaschine füllt man in eine Hartgelatine-Kapsel der Größe 0 long die 250 mg PDE-Hemmer entsprechende Menge Granulat ein und legt das 100 mg Substanz B enthaltende Dragée obenauf.

Beispiel 17

Suspension, enthaltend 10 mg (-)-5E-6-[4-(Z-2-(4-Chlorphenyl)-cyclopropyl-1-carboxamido)-phenyl]-6-(3-pyridyl)-hex-5-ensäure (Substanz B) + 100 mg Dipyridamol pro 5 g.

Die Suspension hat folgende Zusammensetzung:

| | | |
|---|---|---|
| (1) | Dipyridamol | 2,0 % |
| (2) | Substanz B | 0,2 % |
| (3) | Sorbit | 20,8 % |
| (4) | Cellulose | 7,5 % |
| (5) | Natriumcarboxymethylcellulose | 2,5 % |
| (6) | Geschmackskorrigentien/Konservierungsstoffe | 1,8 % |
| (7) | Wasser | 65,2 % |

In heißes Wasser wird unter hoher Scherung (3) - (6) eingerührt. Nach Abkühlung werden in die viskose Suspension (1), (2) und (7) eingearbeitet.

Beispiel 18

Depot-Form, enthaltend 50 mg (-)-5E-6-[4-(Z-2-(4-Chlorphenyl)-cyclopropyl-1-carboxamido)-phenyl]-6-(3-pyridyl)-hex-5-ensäure (Substanz B) + 200 mg Dipyridamol

a) Pellet I

Eine Mischung aus

| | |
|---|---|
| Substanz B | 50,0 kg |
| Lysin | 12,5 kg |
| Hydroxypropylcellulose, hochpolymer | 52,5 kg |
| Triacetin | 4,0 kg |
| Äthylcellulose | 2,5 kg |
| Magnesiumstearat | 3,5 kg |

wird auf einem Spezialextruder mit Äthanol geknetet und in Form von Spaghetti (Durchmesser 1 mm) extrudiert, die in einem Sphäronizer zu Pellets gerundet werden. Danach trocknet man sie gründlich.

b) Pellet II

300 kg ausgemischte Weinsäure-Starterpellets werden in einem Spezialkessel mit einer Suspension bestehend aus Isopropanol, Dipyridamol und Polyvinylpyrrolidon solange besprüht, bis die entstehenden Wirkstoffpellets ca. 45 % Dipyridamol enthalten.

Diese Pellets besprüht man mit einem Lack, der aus Methacrylsäure/Methylmethacrylat-Copolymer (Handelsnahme Eudragit S) und Hydroxypropylmethylcellulosephthalat (Handelsname HP 55) im Gewichtsverhältnis 85:15 bis 50:50 besteht. Die organischen Lacklösungen enthalten noch Weichmacher und Talkum. Es werden zwei Pelletkomponenten mit 5 und 7 % Hüllenmittel und unterschiedlichem Verhältnis der Lackkomponenten in den genannten Grenzen gesprüht. Die beiden Komponenten werden so gemischt, daß sie nachfolgende in vitro-Freigabe ergeben:

Bedingungen (entsprechend USPXXI, Basket-Methode, 100 Umdrehungen/Min.,
1. Stunde künstlicher Magensaft, pH 1,2, 2. bis 6. Stunde künstlicher Darmsaft (Phosphatpuffer), pH 5.5):

Wirkstoff-Freigabe pro Stunde:

| | |
|---|---|
| 1. Stunde | ca. 30 % |
| 2. Stunde | ca. 25 % |
| 3. Stunde | ca. 18 % |
| 4. Stunde | ca. 12 % |

nach der 6. Stunde mehr als 90 % Dipyridamol-Freigabe.

c) Abfüllung

Entsprechend dem Wirkstoffgehalt der Pelletkomponenten I und II und der gewünschten Dosierung werden die Pellets miteinander vermischt und auf einer Kapselmaschine in Kapseln der Größe 0 long abgefüllt.

Beispiel 19

Ampullen, enthaltend 5 mg (-)-5E-6-[4-(Z-2-(4-Chlorphenyl)-cyclopropyl-1-carboxamido)-phenyl]-6-(3-pyridyl)-hex-5-ensäure (Substanz B) + 10 mg Dipyridamol per 5 ml

Zusammensetzung:

| | |
|---|---|
| (1) Dipyridamol | 10 mg |
| (2) Substanz B | 5 mg |
| (3) Propylenglykol | 50 mg |
| (4) Polyethylenglykol | 5 mg |
| (5) Ethanol | 10 mg |
| (6) Wasser für Injektionszwecke ad | 5 ml |
| (7) IN HCl ad | pH 3 |

Die Wirkstoffe werden unter Erwärmung in der Lösung aus (3) - (7) gelöst. Nach pH-Kontrolle und Sterilfiltration füllt man in geeignete Ampullen und sterilisiert.

**Patentansprüche**

**1.** Pyridylderivate der allgemeinen Formel

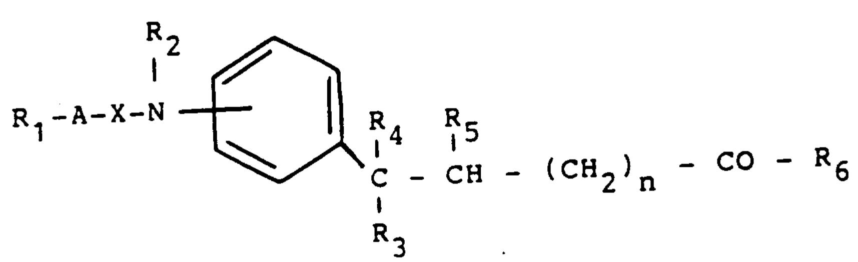

,(I)

in der
n die Zahl 2, 3 oder 4,
X eine Carbonyl- oder Thiocarbonylgruppe,

$R_1$ eine gegebenenfalls durch eine Phenylgruppe substituierte Alkylgruppe mit 1 bis 4 Kohlenstoffatomen, eine Cycloalkylgruppe mit 4 bis 7 Kohlenstoffatomen, eine Naphthyl-, Biphenylyl-, Diphenylmethyl-, Indolyl-, Thienyl-, Chlorthienyl- oder Bromthienylgruppe, eine Phenylgruppe oder auch, wenn A keine Bindung darstellt, eine Benzoylgruppe, in denen der Phenylkern jeweils durch Fluor-, Chlor- oder Bromatome, Alkoxy- oder Alkylgruppen mit 1 bis 4 Kohlenstoffatomen mono-, di- oder trisubstituiert sein kann, wobei die Substituenten gleich oder verschieden sein können und einer der Substituenten auch eine Trifluormethyl-, Carboxyl-, Amino- oder Nitrogruppe darstellen kann,
$R_2$ ein Wasserstoffatom oder eine Alkylgruppe mit 1 bis 4 Kohlenstoffatomen,
$R_3$ eine Pyridylgruppe,
$R_4$ und $R_5$ jeweils ein Wasserstoffatom oder zusammen eine weitere Kohlenstoff-Kohlenstoff-Bindung,
$R_6$ eine Hydroxy- oder Alkoxygruppe mit 1 bis 3 Kohlenstoffatomen und
A eine Bindung, eine Cycloalkylen- oder Cycloalkylidengruppe mit jeweils 3 oder 4 Kohlenstoffatomen, in denen eine Methylengruppe durch eine Dichlormethylengruppe ersetzt sein kann, eine geradkettige gegebenenfalls ein- oder mehrfach ungesättigte Alkylengruppe oder eine Oxyalkylengruppe mit 2 oder 3 Kohlenstoffatomen, eine -$R_7CR_8$-, -O-$R_7CR_8$-, -$NR_9$- oder -$XNR_9$-Gruppe, in denen
X wie eingangs definiert ist,
$R_7$ ein Wasserstoffatom, eine Hydroxy-, Phenyl- oder Alkylgruppe mit 1 bis 3 Kohlenstoffatomen,
$R_8$ ein Wasserstoffatom oder eine Alkylgruppe mit 1 bis 3 Kohlenstoffatomen und
$R_9$ ein Wasserstoffatom, eine Alkylgruppe mit 1 bis 3 Kohlenstoffatomen oder eine Phenylgruppe darstellen,
bedeuten, deren Enantiomere, deren cis- und trans-Isomere, sofern $R_4$ und $R_5$ zusammen eine Kohlenstoff-Kohlenstoff-Bindung darstellen, und deren Additionssalze.

**2.** Pyridylderivate der allgemeinen Formel I gemäß Anspruch 1, in der
n die Zahl 2, 3 oder 4,
X eine Carbonyl- oder Thiocarbonylgruppe,
$R_1$ eine Phenylgruppe oder auch, wenn A keine Bindung darstellt, eine Benzoylgruppe, in denen der Phenylkern jeweils durch ein Fluor-, Chlor- oder Bromatom, durch eine Trifluormethyl-, Phenyl-, Methoxy-, Carboxy- oder Nitrogruppe oder durch eine Alkylgruppe mit 1 bis 4 Kohlenstoffatomen monosubstituiert sein kann, eine Phenylgruppe oder auch, wenn A keine Bindung darstellt, eine Benzoylgruppe, in denen der Phenylkern jeweils durch Chlor- oder Bromatome oder durch Methylgruppen disubstituiert ist, wobei die Substituenten gleich oder verschieden sein können, eine Cyclohexyl-, Benzyl-, 4-Amino-3,5-dichlor-phenyl-, 4-Amino-3,5-dibromphenyl-, Naphthyl-, Diphenylmethyl-, Indolyl-, Thienyl-, Chlorthienyl- oder Bromthienylgruppe,
$R_2$ ein Wasserstoffatom oder eine Alkylgruppe mit 1 bis 3 Kohlenstoffatomen,
$R_3$ eine Pyridylgruppe,
$R_4$ und $R_5$ jeweils ein Wasserstoffatom oder zusammen eine weitere Kohlenstoff-Kohlenstoff-Bindung,
$R_6$ eine Hydroxy- oder Alkoxygruppe mit 1 bis 3 Kohlenstoffatomen und
A eine Bindung, eine Cyclopropylen- oder Cyclopropylidengruppe, in denen eine Methylengruppe durch eine Dichlormethylengruppe ersetzt sein kann, eine geradkettige gegebenenfalls ein- oder mehrfach ungesättigte Alkylengruppe mit 2 Kohlenstoffatomen, eine -$R_7CR_8$-, -O-$R_7CR_8$- oder -$NR_9$-Gruppe, in denen
$R_7$ ein Wasserstoffatom, eine Hydroxy- oder Alkylgruppe mit 1 oder 2 Kohlenstoffatomen,
$R_8$ ein Wasserstoffatom oder eine Alkylgruppe mit 1 oder 2 Kohlenstoffatomen und
$R_9$ ein Wasserstoffatom, eine Alkylgruppe mit 1 oder 2 bis 3 Kohlenstoffatomen oder eine Phenylgruppe darstellen, bedeuten, deren Enantiomere, deren cis- und trans-Isomere, sofern $R_4$ und $R_5$ zusammen eine Kohlenstoff-Kohlenstoff-Bindung bilden, und deren Additionssalze.

**3.** Pyridylderivate der allgemeinen Formel I gemäß Anspruch 1, in der
n die Zahl 2, 3 oder 4,
X eine Carbonyl- oder Thiocarbonylgruppe,
$R_1$ eine gegebenenfalls durch ein Fluor-, Chlor- oder Bromatom, durch eine Trifluormethyl-, Phenyl-, Methoxy-, Carboxy- oder Nitrogruppe oder durch eine Alkylgruppe mit 1 bis 4 Kohlenstoffatomen monosubstituierte Phenylgruppe, eine durch Chlor- oder Bromatome oder durch Methylgruppen disubstituierte Phenylgruppe, wobei die Substituenten gleich oder verschieden sein können, eine Benzyl-, 4-Amino-3,5-dichlorphenyl-, Naphthyl- oder Chlorthienylgruppe,
$R_2$ ein Wasserstoffatom oder eine Methylgruppe,
$R_3$ eine 3-Pyridylgruppe,
$R_4$ und $R_5$ jeweils ein Wasserstoffatom oder zusammen eine weitere Kohlenstoff-Kohlenstoff-Bindung,
$R_6$ eine Hydroxygruppe und
A eine Bindung, eine Cyclopropylen- oder Cyclopropylidengruppe, eine geradkettige gegebenenfalls ein- oder mehrfach ungesättigte Alkylengruppe mit 2 Kohlenstoffatomen, eine -$R_7CR_8$-, -O-$R_7CR_8$- oder -$NR_9$-Gruppe, in denen

$R_7$ ein Wasserstoffatom, eine Hydroxy- oder Methylgruppe,
$R_8$ ein Wasserstoffatom oder eine Methylgruppe und
$R_9$ ein Wasserstoffatom, eine Methyl- oder Phenylgruppe darstellen, bedeuten, deren Enantiomere, deren cis- und trans-Isomere, sofern $R_4$ und $R_5$ zusammen eine Kohlenstoff-Kohlenstoff-Bindung bilden, und deren Additionssalze.

4. Als Verbindungen der Formel I gemäß Anspruch 1:
(-)-5E-6-[4-(Z-2-(4-Chlorphenyl)-cyclopropyl-1-carboxamido)-phenyl]-6-(3-pyridyl)-hex-5-ensäure,
5E-6-[3-(3-(4-Chlorphenyl)-propionylamino)-phenyl]-6-(3-pyridyl)-hex-5-ensäure,
5E-6-[3-(3-(4-Chlorphenyl)-thioureido)-phenyl]-6-(3-pyridyl)-hex-5-ensäure und
5E-6-[3-(3-(2,4-Dichlorphenyl)-1-methylthioureido)-phenyl]-6-(3-pyridyl)-hex-5-ensäure,
deren cis- und trans-Isomere und deren Additionssalze.

5. (-)-5E-6-[4-(Z-2-(4-Chlorphenyl)-cyclopropyl-1-carboxamido)-phenyl]-6-(3-pyridyl)-hex-5-ensäure, dessen cis- und trans-Isomere und dessen Additionssalze.

6. Physiologisch verträgliche Additionssalze der Verbindung gemäß den Ansprüchen 1 bis 5 mit anorganischen oder organischen Basen.

7. Arzneimittel, enthaltend als Wirkstoff eine Verbindung gemäß den Ansprüchen 1 bis 5 oder dessen physiologisch verträgliches Additionssalz gemäß Anspruch 6 neben gegebenenfalls einem oder mehreren inerten Trägerstoffen und/oder Verdünnungsmitteln.

8. Arzneimittel gemäß Anspruch 7 geeignet zur Behandlung und zur Prophylaxe thrombo-embolischer Erkrankungen, zur Prophylaxe der Arteriosklerose und zur Metastasenprophylaxe sowie zur Behandlung der Ischämie, des Asthmas und von Allergien.

9. Arzneimittel gemäß Anspruch 7 geeignet zur Behandlung und zur Prophylaxe von Erkrankungen bei denen eine thromboxanvermittelte Konstriktion oder $PGE_2$ vermittelte Dilatation der Kapillaren eine Rolle spielen, zur Verminderung des Schweregrades einer Transplantatabstoßung, zur Verminderung der renalen Toxizität von Substanzen wie Cyclosporin, zur Behandlung von Nierenerkrankungen und zur Behandlung von Schockzuständen.

10. Arzneimittel gemäß Anspruch 7, 8 oder 9 dadurch gekennzeichnet, daß dieses zusätzlich als Wirkstoff einen PDE-Hemmer oder ein Lysemittel enthalten.

11. Verfahren zur Herstellung eines Arzneimittels gemäß den Ansprüchen 7 bis 10, dadurch gekennzeichnet, daß auf nichtchemischem Wege eine Verbindung gemäß den Ansprüchen 1 bis 5 oder dessen physiologisch verträgliches Additionssalz gemäß Anspruch 6 gegebenenfalls in Kombination mit einem PDE-Hemmer oder einem Lysemittel in einen oder mehrere inerte übliche Trägerstoffe und/oder Verdünnungsmittel eingearbeitet wird.

12. Verfahren zur Herstellung der Verbindungen gemäß den Ansprüchen 1 bis 6, dadurch gekennzeichnet, daß

a) eine Verbindung der allgemeinen Formel

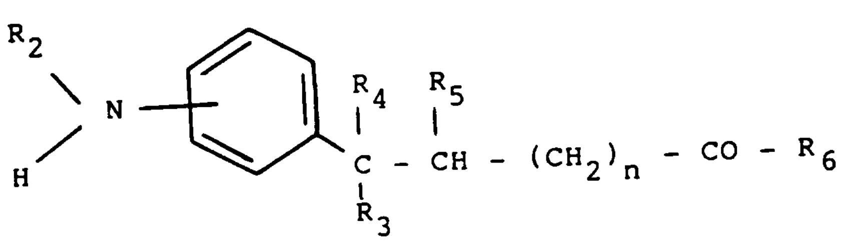

,(II)

in der
$R_2$ bis $R_6$ und n wie in den Ansprüchen 1 bis 5 definiert sind, mit einer Verbindung der allgemeinen Formel

$$R_1 - A - X - Z_1 \quad ,(III)$$

in der
$R_1$ wie in den Ansprüchen 1 bis 5 definiert ist und
$Z_1$ eine nucleophile Austrittsgruppe oder auch, wenn A eine $-NR_9$-Gruppe und X eine Carbonyl- oder Thiocarbonylgruppe darstellen, $Z_1$ zusammen mit $R_9$ eine weitere Kohlenstoff-Stickstoff-Bindung darstellt, acyliert wird oder

b) zur Herstellung von Verbindungen der allgemeinen Formel I, in der $R_6$ eine Hydroxygruppe darstellt, ein Schutzrest von einer Verbindung der allgemeinen Formel

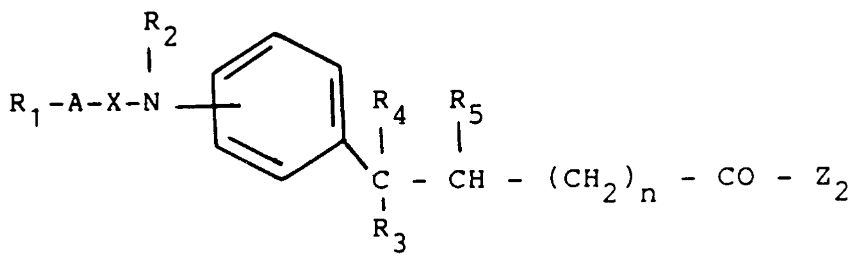

,(IV)

in der
$R_1$ bis $R_5$, A, X und n wie in den Ansprüchen 1 bis 5 definiert sind und
$Z_2$ eine hydrolytisch, thermolytisch oder hydrogenolytisch abspaltbare Schutzgruppe für eine Carboxygruppe oder ein funktionelles Derivat der Carboxygruppe darstellt, abgespalten wird oder

c) zur Herstellung von Verbindungen der allgemeinen Formel I, in der $R_4$ und $R_5$ jeweils ein Wasserstoffatom darstellen, eine Verbindung der allgemeinen Formel

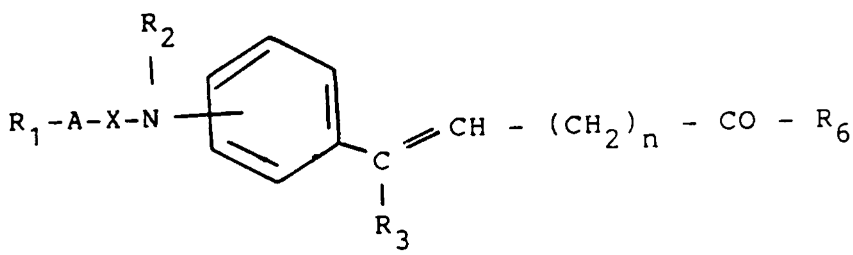

,(V)

in der
$R_1$ bis $R_3$, $R_6$, A, X und n wie in den Ansprüchen 1 bis 5 definiert sind, hydriert wird oder

d) zur Herstellung von Verbindungen der allgemeinen Formel I, in der $R_4$ und $R_5$ zusammen eine Kohlenstoff-Kohlenstoff-Bindung darstellen, eine Verbindung der allgemeinen Formel

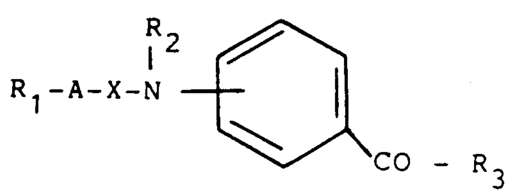

,(VI)

in der
$R_1$ bis $R_3$, A und X wie in den Ansprüchen 1 bis 5 definiert sind, mit einer Verbindung der allgemeinen Formel

$$W - CH_2 - (CH_2)_n - CO - R_6 \quad ,(VII)$$

in der
$R_6$ und n wie in den Ansprüchen 1 bis 5 definiert sind und
W einen Triphenylphosphoniumhalogenid-, Dialkylphosphonsäure- oder ein Magnesiumhalogenidrest bedeuten, umgesetzt und gegebenenfalls anschließend dehydratisiert wird und
gewünschtenfalls anschließend eine so erhaltene Verbindung der Formel I, in der $R_2$ ein Wasserstoffatom darstellt, mittels Alkylierung in eine entsprechende Verbindung der Formel I, in der $R_2$ eine Alkylgruppe darstellt, übergeführt wird oder
eine so erhaltene Verbindung der Formel I, in der $R_6$ eine Hydroxygruppe darstellt, mittels Veresterung in eine entsprechende Verbindung der Formel I, in der $R_6$ eine Alkoxy-, Amino-, Alkylamino- oder Dialkylaminogruppe darstellt, übergeführt wird oder
eine so erhaltene Verbindung der Formel I, in der $R_4$ und $R_5$ zusammen eine Kohlenstoff-Kohlenstoff-Bindung darstellen, in ihre cis- und trans-Isomere aufgetrennt wird oder
eine so erhaltene Verbindung der Formel I in ihre Enantiomeren aufgetrennt wird oder
eine so erhaltene Verbindung der Formel I in ihre Additionssalze, insbesondere in ihre physiologisch verträglichen Additionssalze mit anorganischen oder organischen Basen übergeführt wird.

## Claims

**1.** Pyridyl derivatives of general formula

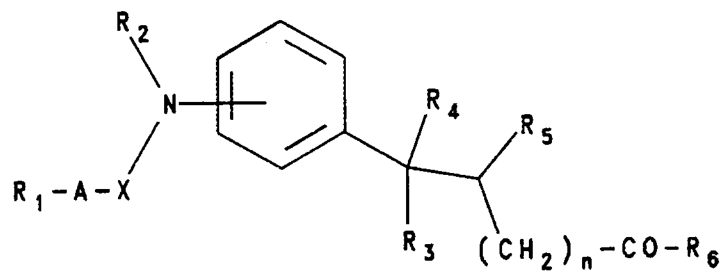

(I)

wherein
n represents the number 2, 3 or 4,
X represents a carbonyl or thiocarbonyl group,
$R_1$ represents an optionally phenyl-substituted $C_{1-4}$-alkyl group, a $C_{4-7}$-cycloalkyl group, a naphthyl, biphenylyl, diphenylmethyl, indolyl, thienyl, chlorothienyl or bromothienyl group, a phenyl group or, if A does not represent a bond, $R_1$ may represent a benzoyl group in which the phenyl nucleus may in each case be mono-, di-or trisubstituted by fluorine, chlorine or bromine atoms or by $C_{1-4}$-alkoxy or $C_{1-4}$-alkyl groups these substituents being either identical or different and one of the substituents may also represent a trifluoromethyl, carboxyl, amino or nitro group,
$R_2$ represents a hydrogen atom or a $C_{1-4}$-alkyl group,
$R_3$ represents a pyridyl group,
$R_4$ and $R_5$ each represent a hydrogen atom or together represent another carbon-carbon bond,
$R_6$ represents a hydroxy or $C_{1-3}$-alkoxy group, and
A represents a bond, a $C_{3-4}$-cycloalkylene or $C_{3-4}$-cycloalkylidene group wherein a methylene group may be replaced by a dichloromethylene group, or A represents a straight-chained, optionally mono- or polyunsaturated alkylene group or a $C_{2-3}$-oxyalkylene group, an $-R_7CR_8-$, $-O-R_7CR_8-$, $NR_9-$ or $-XNR_9$ group, wherein
X is as hereinbefore defined,
$R_7$ represents a hydrogen atom, a hydroxy, phenyl or $C_{1-3}$-alkyl group,
$R_8$ represents a hydrogen atom or a $C_{1-3}$-alkyl group and
$R_9$ represents a hydrogen atom, a $C_{1-3}$-alkyl group or a phenyl group,
the enantiomers, the cis- and trans-isomers if $R_4$ and $R_5$ together represent a carbon-carbon bond, and the addition salts thereof.

**2.** Pyridyl derivatives of general formula I according to claim 1, wherein
n represents the number 2, 3 or 4,
X represents a carbonyl or thiocarbonyl group,
$R_1$ represents a phenyl group or, if A does not represent a bond, $R_1$ may also represent a benzoyl group in which the phenyl nucleus may be monosubstituted in each case by a fluorine, chlorine or bromine atom, by a trifluoromethyl, phenyl, methoxy, carboxy or nitro group or by a $C_{1-4}$-alkyl group, or $R_1$ may represent a phenyl group or, if A does not represent a bond, $R_1$ may also represent a benzoyl group in which the phenyl nucleus in each case is disubstituted by chlorine or bromine atoms or by methyl groups these substituents being either identical or different, or $R_1$ may

represent a cyclohexyl, benzyl, 4-amino-3,5-dichlorophenyl, 4-amino-3,5-dibromophenyl, naphthyl, diphenylmethyl, indolyl, thienyl, chlorothienyl or bromothienyl group,
$R_2$ represents a hydrogen atom or a $C_{1-3}$-alkyl group,
$R_3$ represents a pyridyl group,
$R_4$ and $R_5$ each represent a hydrogen atom or together represent another carbon-carbon bond,
$R_6$ represents a hydroxy or $C_{1-3}$-alkoxy group and
A represents a bond, a cyclopropylene or cyclopropylidene group wherein a methylene group may be replaced by a dichloromethylene group, a straight-chained, optionally mono- or polyunsaturated alkylene group having 2 carbon atoms, or an $-R_7CR_8-$, $-O-R_7CR_8-$ or $-NR_9-$ group,
wherein
$R_7$ represents a hydrogen atom, a hydroxy or a $C_{1-2}$-alkyl group,
$R_8$ represents a hydrogen atom or a $C_{1-2}$-alkyl group and
$R_9$ represents a hydrogen atom, an alkyl group having 1 or 2 to 3 carbon atoms or a phenyl group,
the enantiomers thereof, the cis- and trans-isomers thereof if $R_4$ and $R_5$ together represent a carbon-carbon bond, and the addition salts thereof.

**3.** Pyridyl derivatives of general formula I according to claim 1, wherein
n represents the number 2, 3 or 4,
X represents a carbonyl or thiocarbonyl group,
$R_1$ represents a phenyl group optionally monosubstituted by a fluorine, chlorine or bromine atom, by a trifluoromethyl, phenyl, methoxy, carboxy or nitro group or by a $C_{1-4}$-alkyl group, or $R_1$ represents a phenyl group disubstituted by chlorine or bromine atoms or methyl groups these substituents being either identical or different, or $R_1$ represents a benzyl, 4-amino-3,5-dichlorophenyl, naphthyl or chlorothienyl group,
$R_2$ represents a hydrogen atom or a methyl group,
$R_3$ represents a 3-pyridyl group,
$R_4$ and $R_5$ each represent a hydrogen atom or together represent another carbon-carbon bond,
$R_6$ represents a hydroxy group and
A represents a bond, a cyclopropylene or cyclopropylidene group, a straight-chained, optionally mono- or polyunsaturated $C_2$-alkylene group, an $-R_7CR_8-$, $-O-R_7CR_8-$ or $-NR_9-$ group, wherein
$R_7$ represents a hydrogen atom or a hydroxy or methyl group,
$R_8$ represents a hydrogen atom or a methyl group and
$R_9$ represents a hydrogen atom or a methyl or phenyl group,
the enantiomers thereof, the cis- and trans-isomers thereof if $R_4$ and $R_5$ together form a carbon-carbon bond, and the addition salts thereof.

**4.** As compounds of formula I according to claim 1:
(-)-5E-6-[4-(Z-2-(4-chlorophenyl)-cyclopropyl-1-carboxamido)-phenyl]-6-(3-pyridyl)-hex-5-enoic acid,
5E-6-[3-(3-(4-chlorophenyl)-propionylamino)-phenyl]-6-(3-pyridyl)-hex-5-enoic acid,
5E-6-[3-(3-(4-chlorophenyl)-thioureido)-phenyl]-6-(3-pyridyl)-hex-5-enoic acid and
5E-6-[3-(3-(2,4-dichlorophenyl)-1-methylthioureido)-phenyl]-6-(3-pyridyl)-hex-5-enoic acid,
the cis- and trans-isomers thereof and the addition salts thereof.

**5.** (-)-5E-6-[4-(Z-2-(4-chlorophenyl)-cyclopropyl-1-carboxamido)-phenyl]-6-(3-pyridyl)-hex-5-enoic acid, the cis- and trans-isomers thereof and the addition salts thereof.

**6.** Physiologically acceptable addition salts of the compound according to claims 1 to 5 with inorganic or organic bases.

**7.** Pharmaceutical compositions containing as active substance a compound according to claims 1 to 5 or a physiologically acceptable addition salt thereof according to claim 6, optionally together with one or more inert carriers and/or diluents.

**8.** Pharmaceutical composition according to claim 7, suitable for the treatment and prevention of thromboembolic disorders, for the prevention of arteriosclerosis and metastasis and for the treatment of ischaemia, asthma and allergies.

**9.** Pharmaceutical composition according to claim 7, suitable for the treatment and prevention of diseases in which a thromboxane-mediated constriction or $PGE_2$-mediated dilation of the capillaries is involved, for reducing the severity of transplant rejection, for reducing the renal toxicity of substances such as cyclosporin, for the treatment of kidney diseases and for the treatment of states of shock.

**10.** Pharmaceutical composition according to claim 7, 8 or 9, characterised in that it additionally contains as active substance a PDE-inhibitor or a lysing agent.

**11.** Process for preparing a pharmaceutical composition according to claims 7 to 10, characterised in that a compound according to claims 1 to 5 or a physiologically acceptable addition salt thereof according to claim 6, optionally in conjunction with a PDE-inhibitor or a lysing agent, is incorporated, by a non-chemical method, in one or more inert conventional carriers and/or diluents.

**12.** Process for preparing compounds according to claims 1 to 6, characterised in that

a) a compound of general formula

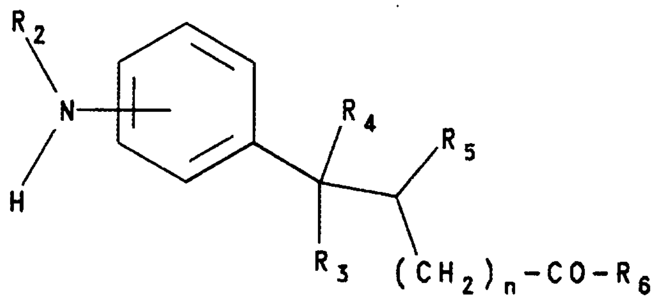

(II)

wherein
$R_2$ to $R_6$ and n are defined as in claims 1 to 5, is acylated with a compound of general formula

$$R_1 - A - X - Z_1 \qquad (III)$$

wherein
$R_1$ is defined as in claims 1 to 5 and
$Z_1$ represents a nucleophilic leaving group or, if A represents an $-NR_9-$ group and X represents a carbonyl or thiocarbonyl group, $Z_1$ together with $R_9$ represents another carbon-nitrogen bond, or

b) in order to prepare compounds of general formula I wherein $R_6$ represents a hydroxy group, a protecting group is cleaved from a compound of general formula

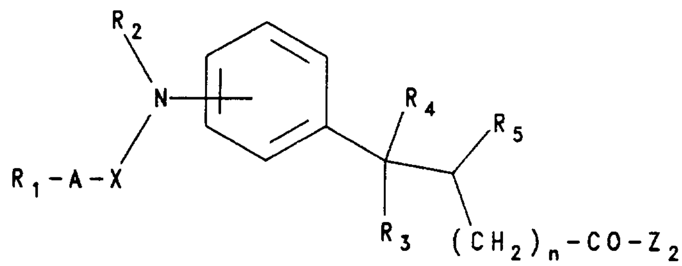

(IV)

wherein
$R_1$ to $R_5$, A, X and n are defined as in claims 1 to 5 and $Z_2$ represents a hydrolytically, thermolytically or hydrogenolytically cleavable protecting group for a carboxy group or a functional derivative of the carboxy group, or

c) in order to prepare compounds of general formula I wherein $R_4$ and $R_5$ each represent a hydrogen atom, a compound of general formula

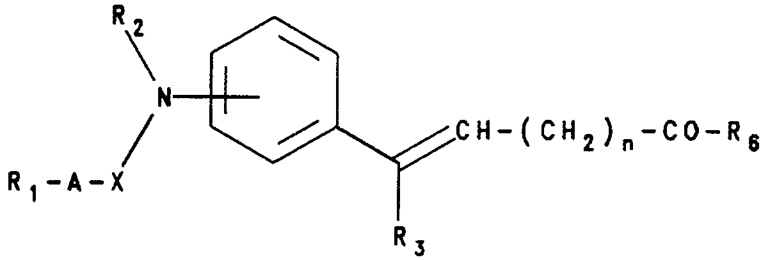

(V)

wherein
$R_1$ to $R_3$, $R_6$, A, X and n are defined as in claims 1 to 5, is hydrogenated, or

d) in order to prepare compounds of general formula I wherein $R_4$ and $R_5$ together represent a carbon-carbon bond, a compound of general formula

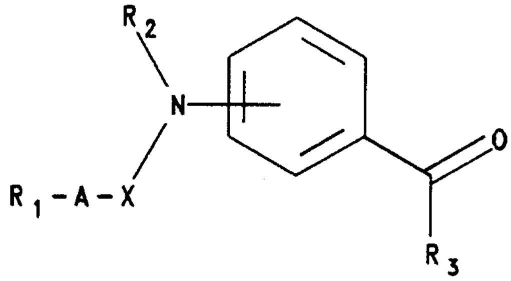

(VI)

wherein
$R_1$ to $R_3$, A and X are defined as in claims 1 to 5, is reacted with a compound of general formula

$$W - CH_2 - (CH_2)_n - CO - R_6 \qquad (VII)$$

wherein
$R_6$ and n are defined as in claims 1 to 5 and
W represents a triphenylphosphonium halide, dialkylphosphonic acid or magnesium halide group, and if desired subsequently dehydrated and
if desired a compound of formula I thus obtained wherein $R_2$ represents a hydrogen atom may subsequently be converted by alkylation into a corresponding compound of formula I wherein $R_2$ represents an alkyl group, or
a compound of formula I thus obtained wherein $R_6$ represents a hydroxy group may be converted by esterification into a corresponding compound of formula I wherein $R_6$ represents an alkoxy, amino, alkylamino or dialkylamino group, or
a compound of formula I thus obtained wherein $R_4$ and $R_5$ together represent a carbon-carbon bond is resolved into the cis- and trans-isomers thereof or
a compound of formula I thus obtained is resolved into the enantiomers thereof or
a compound of formula I thus obtained is converted into the addition salts thereof, more particularly the physiologically acceptable addition salts thereof with inorganic or organic bases.

## Revendications

1. Dérivés pyridyliques de formule générale

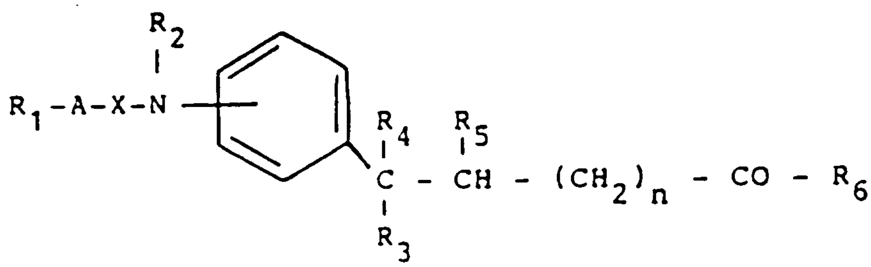

(I)

dans laquelle
n représente le nombre 2, 3 ou 4,
X représente un groupe carbonyle ou thiocarbonyle,
$R_1$ représente un groupe alkyle de 1 à 4 atomes de carbone éventuellement substitué par un groupe phényle, un groupe cycloalkyle de 4 à 7 atomes de carbone, un groupe naphtyle, biphénylyle, diphénylméthyle, indolyle, thiényle, chlorothiényle ou bromothiényle, un groupe phényle ou encore, lorsque A ne représente pas une liaison, un groupe benzoyle, dans lesquels le noyau phényle peut être mono-, di- ou trisubstitué dans chaque cas par des atomes de fluor, de chlore ou de brome, des groupes alcoxy ou alkyle de 1 à 4 atomes de carbone, les substituants pouvant être identiques ou différents et l'un des substituants pouvant aussi représenter un groupe trifluorométhyle, carboxyle, amino ou nitro,
$R_2$ représente un atome d'hydrogène ou un groupe alkyle de 1 à 4 atomes de carbone,
$R_3$ représente un groupe pyridyle,
$R_4$ et $R_5$ représentent chacun un atome d'hydrogène ou ensemble une autre liaison carbone-carbone,
$R_6$ représente un groupe hydroxyle ou alcoxy de 1 à 3 atomes de carbone et
A représente une liaison, un groupe cycloalkylène ou cycloalkylidène de 3 ou 4 atomes de carbone dans chaque cas, dans lesquels un groupe méthylène peut être remplacé par un groupe dichlorométhylène, un groupe alkylène linéaire éventuellement insaturé une ou plusieurs fois ou un groupe oxyalkylène de 2 ou 3 atomes de carbone, un groupe $-R_7CR_8-$, $-O-R_7CR_8-$, $-NR_9-$ ou $-XNR_9-$, dans lesquels
    X est défini comme au début,
    $R_7$ représente un atome d'hydrogène, un groupe hydroxyle, phényle ou alkyle de 1 à 3 atomes de carbone,
    $R_8$ représente un atome d'hydrogène ou un groupe alkyle de 1 à 3 atomes de carbone et
    $R_9$ représente un atome d'hydrogène, un groupe alkyle de 1 à 3 atomes de carbone ou un groupe phényle,
leurs énantiomères, leurs isomères cis et trans, dans la mesure où $R_4$ et $R_5$ représentent ensemble une liaison carbone-carbone, et leurs sels d'addition.

2. Dérivés pyridyliques de formule générale I selon la revendication 1,
dans laquelle
n représente le nombre 2, 3 ou 4,
X représente un groupe carbonyle ou thiocarbonyle,
$R_1$ représente un groupe phényle ou encore, lorsque A ne représente pas une liaison, un groupe benzoyle, dans lesquels le noyau phényle peut être monosubstitué dans chaque cas par un atome de fluor, de chlore ou de brome, par un groupe trifluorométhyle, phényle, méthoxy, carboxyle ou nitro ou par un groupe alkyle de 1 à 4 atomes de carbone, un groupe phényle, ou encore, lorsque A ne représente pas une liaison, un groupe benzoyle, dans lesquels le noyau phényle est disubstitué dans chaque cas par des atomes de chlore ou de brome ou par des groupes méthyle, les substituants pouvant être identiques ou différents, un groupe cyclohexyle, benzyle, 4-amino-3,5-dichlorophényle, 4-amino-3,5-dibromophényle, naphtyle, diphénylméthyle, indolyle, thiényle, chlorothiényle ou bromothiényle,
$R_2$ représente un atome d'hydrogène ou un groupe alkyle de 1 à 3 atomes de carbone,
$R_3$ représente un groupe pyridyle,
$R_4$ et $R_5$ représentent chacun un atome d'hydrogène ou ensemble une autre liaison carbone-carbone,
$R_6$ représente un groupe hydroxyle ou alcoxy de 1 à 3 atomes de carbone et
A représente une liaison, un groupe cyclopropylène ou cyclopropylidène, dans lesquels un groupe méthylène peut être remplacé par un groupe dichlorométhylène, un groupe alkylène linéaire de 2 atomes de carbone éventuellement insaturé une ou plusieurs fois, un groupe $-R_7CR_8-$, $-O-R_7CR_8-$ou $-NR_9-$ dans lesquels

$R_7$ représente un atome d'hydrogène, un groupe hydroxyle ou alkyle de 1 ou 2 atomes de carbone,
$R_8$ représente un atome d'hydrogène ou un groupe alkyle de 1 ou 2 atomes de carbone et
$R_9$ représente un atome d'hydrogène, un groupe alkyle de 1 ou 2 à 3 atomes de carbone ou un groupe phényle,
leurs énantiomères, leurs isomères cis et trans, dans la mesure où $R_4$ et $R_5$ représentent ensemble une liaison carbone-carbone, et leurs sels d'addition.

3. Dérivés pyridyliques de formule générale I selon la revendication 1,
dans laquelle
n représente le nombre 2, 3 ou 4,
X représente un groupe carbonyle ou thiocarbonyle,
$R_1$ représente un groupe phényle éventuellement monosubstitué par un atome de fluor, de chlore ou de brome, par un groupe trifluorométhyle, phényle, méthoxy, carboxyle ou nitro ou par un groupe alkyle de 1 à 4 atomes de carbone, un groupe phényle disubstitué par des atomes de chlore ou de brome ou par des groupes méthyle, les substituants pouvant être identiques ou différents, un groupe benzyle, 4-amino-3,5-dichlorophényle, naphtyle ou chlorothiényle,
$R_2$ représente un atome d'hydrogène ou un groupe méthyle,
$R_3$ représente un groupe 3-pyridyle,
$R_4$ et $R_5$ représentent chacun un atome d'hydrogène ou ensemble une autre liaison carbone-carbone,
$R_6$ représente un groupe hydroxyle et
A représente une liaison, un groupe cyclopropylène ou cyclopropylidène, un groupe alkylène linéaire de 2 atomes de carbone éventuellement insaturé une ou plusieurs fois, un groupe $-R_7CR_8-$, $-O-R_7CR_8-$ ou $-NR_9-$ dans lesquels
$R_7$ représente un atome d'hydrogène, un groupe hydroxyle ou méthyle,
$R_8$ représente un atome d'hydrogène ou un groupe méthyle et
$R_9$ représente un atome d'hydrogène, un groupe méthyle ou phényle,
leurs énantiomères, leurs isomères cis et trans, dans la mesure où $R_4$ et $R_5$ représentent ensemble une liaison carbone-carbone, et leurs sels d'addition.

4. En tant que composés de formule I selon la revendication 1:
acide (-)-5E-6-[4-(Z-2-(4-chlorophényl)-cyclopropyl-1-carboxamido)-phényl]-6-(3-pyridyl)-hex-5-énoïque,
acide 5E-6-[3-(3-(4-chlorophényl)-propionylamino)-phényl]-6-(3-pyridyl)-hex-5-énoïque,
acide 5E-6-[3-(3-(4-chlorophényl)-thiouréido)-phényl]-6-(3-pyridyl)-hex-5-énoïque et
acide 5E-6-[3-(3-(2,4-dichlorophényl)-1-méthylthiouréido)-phényl]-6-(3-pyridyl)-hex-5-énoïque,
leurs isomères cis et trans et leurs sels d'addition.

5. Acide (-)-5E-6-[4-(Z-2-(4-chlorophényl)-cyclopropyl-1-carboxamido)-phényl]-6-(3-pyridyl)-hex-5-énoïque, ses isomères cis et trans et ses sels d'addition.

6. Sels d'addition physiologiquement acceptables du composé selon les revendications 1 à 5 avec des bases inorganiques ou organiques.

7. Médicament contenant comme principe actif un composé selon les revendications 1 à 5 ou son sel d'addition physiologiquement acceptable selon la revendication 6 et éventuellement un ou plusieurs supports et/ou diluants inertes.

8. Médicament selon la revendication 7 qui convient pour le traitement ou pour la prophylaxie des maladies thromboemboliques, pour la prophylaxie de l'artériosclérose et pour la prophylaxie des métastases ainsi que pour le traitement de l'ischémie, de l'asthme et des allergies.

9. Médicament selon la revendication 7 qui convient pour le traitement et pour la prophylaxie des maladies dans lesquelles une constriction des capillaires provoquée par le thromboxane ou une dilatation des capillaires provoquée par $PGE_2$ joue un rôle, pour réduire le degré de gravité d'un rejet de greffe, pour réduire la toxicité rénale de substances comme la cyclosporine, pour le traitement des maladies rénales et pour le traitement des états de choc.

10. Médicament selon la revendication 7, 8 ou 9 caractérisé en ce qu'il contient en outre comme principe actif un inhibiteur de PDE ou un agent de lyse.

11. Procédé de préparation d'un médicament selon les revendications 7 à 10, caractérisé en ce que, par voie non chimique, un composé selon les revendications 1 à 5 ou son sel d'addition physiologiquement acceptable selon la

revendication 6 éventuellement en combinaison avec un inhibiteur de PDE ou un agent de lyse est incorporé dans un ou plusieurs supports et/ou diluants inertes habituels.

**12.** Procédé de préparation des composés selon les revendications 1 à 6, caractérisé en ce que

a) un composé de formule générale

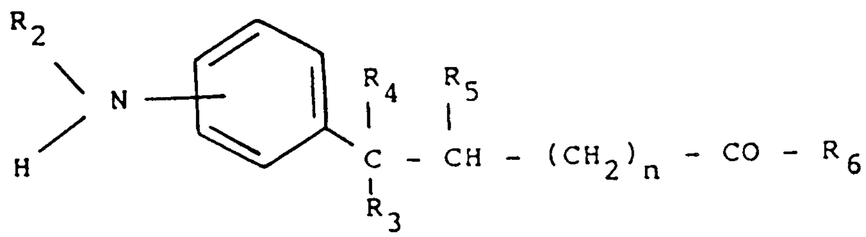

(II)

dans laquelle
$R_2$ à $R_6$ et n sont définis comme dans les revendications 1 à 5 est acylé avec un composé de formule générale

$$R_1 - A - X - Z_1 \qquad \text{(III)}$$

dans laquelle
$R_1$ est défini comme dans les revendications 1 à 5 et
$Z_1$ représente un groupe partant nucléophile ou encore, lorsque A représente un groupe $-NR_9-$ et X représente un groupe carbonyle ou thiocarbonyle, $Z_1$ représente avec $R_9$ une autre liaison carbone-azote, ou
b) pour la préparation de composés de formule générale I dans laquelle $R_6$ représente un groupe hydroxyle, un reste protecteur d'un composé de formule générale

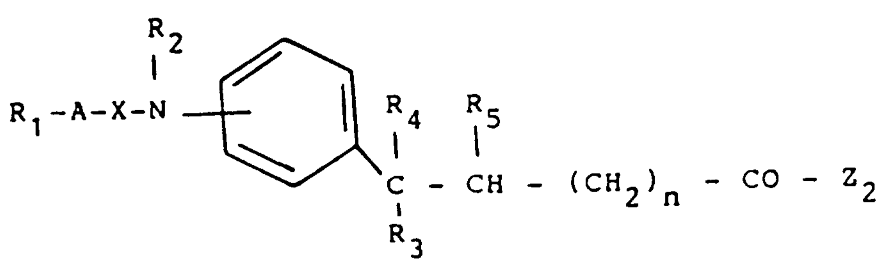

(IV)

dans laquelle
$R_1$ à $R_5$, A, X et n sont définis comme dans les revendications 1 à 5 et
$Z_2$ représente un groupe protecteur clivable par hydrolyse, thermolyse ou hydrogénolyse pour un groupe carboxyle ou un dérivé fonctionnel du groupe carboxyle est clivé ou
c) pour la préparation de composés de formule générale I dans laquelle $R_4$ et $R_5$ représentent chacun un atome d'hydrogène, un composé de formule générale

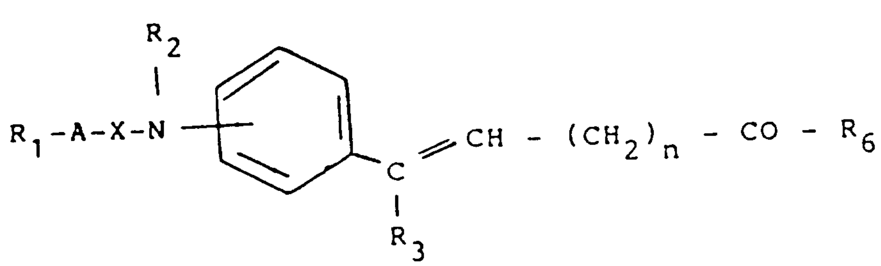

(V)

dans laquelle
$R_1$ à $R_3$, $R_6$, A, X et n sont définis comme dans les revendications 1 à 5, est hydrogéné ou
d) pour la préparation de composés de formule générale I dans laquelle $R_4$ et $R_5$ représentent ensemble une liaison carbone-carbone, un composé de formule générale

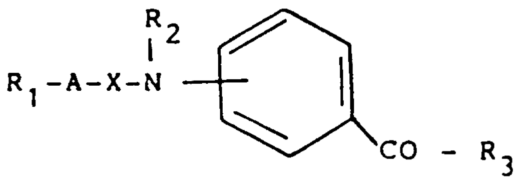

(VI)

dans laquelle
$R_1$ à $R_3$, A et X sont définis comme dans les revendications 1 à 5 est mis à réagir avec un composé de formule générale

$$W - CH_2 - (CH_2)_n - CO - R_6 \qquad (VII)$$

dans laquelle
$R_6$ et n sont définis comme dans les revendications 1 à 5 et W représente un reste halogénure de triphénylphosphonium, acide dialkylphosphonique ou halogénure de magnésium, puis est éventuellement déshydraté et si on le souhaite, un composé ainsi obtenu de formule I dans laquelle $R_2$ représente un atome d'hydrogène est ensuite converti par alkylation en un composé correspondant de formule I dans laquelle $R_2$ représente un groupe alkyle, ou un composé ainsi obtenu de formule I dans laquelle $R_6$ représente un groupe hydroxyle est converti par estérification en un composé correspondant de formule I dans laquelle $R_6$ représente un groupe alcoxy, amino, alkylamino ou dialkylamino, ou
un composé ainsi obtenu de formule I dans laquelle $R_4$ et $R_5$ représentent ensemble une liaison carbone-carbone est résolu en ses isomères cis et trans ou
un composé ainsi obtenu de formule I est résolu en ses énantiomères ou
un composé ainsi obtenu de formule I est converti en ses sels d'addition, en particulier en ses sels d'addition physiologiquement acceptables avec des bases inorganiques ou organiques.